# EUROPEAN PATENT SPECIFICATION

(11) **EP 0 552 379 B1**
(45) Date of publication and mention of the grant of the patent: **30.10.1996**
(21) Application number: 92916962.1
(22) Date of filing: 10.08.1992
(51) Int. Cl.: C07D 211/90, C07D 295/096, A61K 31/44, A61K 31/495

(54) **DIHYDROPYRIDINE DERIVATIVES, AND PROCESS FOR THEIR PREPARATION**
DIHYDROPYRIDINDERIVATE UND VERFAHREN ZU IHRER HERSTELLUNG
DERIVES DE DIHYDROPYRIDINE ET PROCEDE POUR LEUR PREPARATION

(30) Priority: 10.08.1991 GB 9117340
(43) Date of publication of application: 28.07.1993
(73) Proprietor: FUJISAWA PHARMACEUTICAL CO., LTD., Osaka-shi Osaka 541 (JP); DAICEL CHEMICAL INDUSTRIES, LTD., Sakai-shi Osaka-fu 590 (JP)
(72) Inventor: ITO, Kiyotaka Lions Mansion Akashihigashifutami207, Akashi-shi Hyogo 674 (JP); AKAMATSU, Hidekazu, Tsukuba-shi Ibaraki 305 (JP); INOUE, Keizo, Tsukuba-shi Ibaraki 300-32 (JP); ONOMURA, Osamu, Tsukuba-shi Ibaraki 305 (JP); HAMATANI, Takeshi, Tsukuba-shi Ibaraki 305 (JP); UEDA, Yoichiro, Tsukuba-shi Ibaraki 300-32 (JP); ESUMI, Kimio, Kobe-shi Hyogo 658 (JP)
(74) Representative: W.P. THOMPSON & CO.
(86) International application number: JP9201023
(87) International publication number: WO9303014

(56) References cited:
- EP-A- 0 194 046
- EP-A- 0 194 048
- WO-A-86/02640
- DE-A- 3 531 498
- JOURNAL OF MEDICINAL CHEMISTRY vol. 24, no. 5, 1981, pages 628 - 631;

## Description

This invention relates to new dihydropyridine derivatives which possess antihypertensive properties.

Dihydropyridine derivatives and their pharmaceutical activities are disclosed in EP-A-0194046, WO-A-86/02640, DE-A-3531498 and EP-A-0194048 and by J J Baldwin et al in J. Med. Chem. 24 (59) 628-631 (1981).

According to the present invention there is provided a dihydropyridine of the formula: 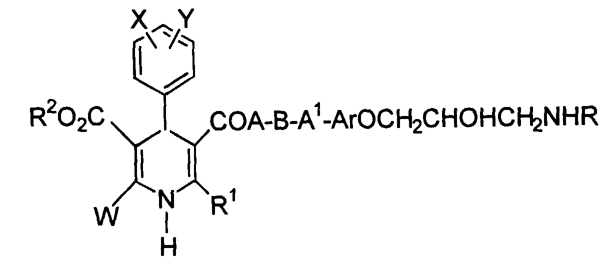 wherein W represents a cyano or halo alkyl group the alkyl group of which is straight chained or branched and contains 1 to 6 carbon atoms; R, R¹ and R², which may be the same or different, each represent a saturated hydrocarbon group which is straight chained or branched and which contains 1 to 6 carbon atoms; X and Y, which may be the same or different, each represents a hydrogen atom, a halogen atom, a halo alkyl group in which the alkyl group is straight chained or branched and contains 1 to 6 carbon atoms, or a nitro group; Ar represents a phenylene group which may contain a methoxy substituent; A represents -0- or -NH-; B represents a direct link or a straight chained or branched bivalent paraffinic hydrocarbon residue of 1 to 10 carbon atoms and A¹ represents a direct link, oxa, imino, an alkylimino having 1 to 6 carbon atoms, amido, an alkylamido having 1 to 6 carbon atoms, oxyalkylenoxy group, a lower or higher straight chained or branched bivalent paraffinic hydrocarbon residue having respectively 2 to 6 or 7 to 12 carbon atoms optionally interrupted by oxa, imino, an alkylimino having 1 to 6 carbon atoms, amido, an alkylamido having 1 to 6 carbon atoms or a saturated or unsaturated, 3 to 8-membered heteromonocylic group containing 1 to 4 nitrogen atoms and optionally 1 or 2 oxygen atoms, or a group of the formula; -Het-Q- (wherein Het is a bivalent, saturated or unsaturated, 3 to 8-membered heteromonocyclic group containing 1 to 4 nitrogen atoms and optionally 1 or 2 oxygen atoms and Q is a direct link or a straight-chained or branched bivalent paraffinic hydrocarbon residue having 2 to 6 carbon atoms); or acid addition salts thereof.

In this specification the term lower alkyl group means a saturated hydrocarbon grouping which is straight-chained or branched and which contains 1 to 6 carbon atoms. Halogen atoms associated with W, X and/or Y are, for example, fluorine, chlorine or bromine, preferably fluorine or chlorine. The term alkylene group of 1 to 10 carbon atoms means a straight-chained or branched bivalent paraffinic hydrocarbon residue of 1 to 10 carbon atoms.

Suitable "lower or higher alkylene group" means a straight-chained or branched bivalent paraffinic hydrocarbon residue of 2 to 6 or 7 to 12, preferably 2 to 6 or 7 to 10 carbon atoms, respectively.

The alkylimino moiety which A¹ may represent is preferably of 1 to 4 carbon atoms.

The alkylimino moiety which may optionally interrupt the lower or higher alkylene group is preferably of 1 to 4 carbon atoms.

The alkylamido moiety which A¹ may represent is preferably of 1 to 4 carbon atoms.

The alkylamido moiety which may optionally interrupt the lower or higher alkylene group is preferably of 1 to 4 carbon atoms.

The heteromonocyclic group moiety which may optionally interrupt the lower or higher alkylene group and/or the bivalent heterocyclic group moiety of Het is preferably a 5 or 6 membered heteromonocyclic group, for example, pyrrolediyl, pyrrolinediyl, imidazolediyl, imidazolinediyl (e.g. 2-imidazolinediyl, etc), pyrazolediyl, pyrazolinediyl, pyridinediyl, pyridinediyl N-oxide, pyridinediylio, dihydropyridinediyl, tetrahydropyridinediyl (e.g. 1,2,3,6-tetrahydropyridinediyl, etc.), pyrimidinediyl, pyrimidinediylio, pyrazinediyl, pyrazinediylio, pyridazinediyl, pyridazinediylio, triazinediyl (e.g. 1,3,5-triazinediyl, 1,2,4-triazinediyl, and 1,2,3-triazinediyl), tetrahydrotriazinediyl (e.g. 1,2,5,6-tetrahydro-1,2,4-triazinediyl, 1,4,5,6-tetrahydro-1,2,4-triazinediyl, etc), triazolediylio, triazolediyl (e.g. 1H-1,2,4-triazolediyl, 1H-1,2,3-triazolediyl, 2H-1,2,3-triazolediyl, etc), tetrazinediyl, tetrazolediyl, (e.g. 1H-tetrazolediyl, 2H-tetrazolediyl), tetrazolediylio, pyrrolidinediyl, inidazolidinediyl, pyrazolidinediyl, piperidinediyl, piperazinediyl, morpholinediyl, etc. Most preferably said heterocyclic group moiety is pyrrolidinediyl, piperidinediyl or piperazinediyl.

Preferably the "lower or higher alkylene group optionally interrupted by oxa, imino, substituted imino or a heterocyclic group" is an oxaalkylene of 2 to 6 carbon atoms, azaalkylene of 2 to 6 carbon atoms, N-(alkyl of 1 to 6 carbon atoms)azaalkylene of 2 to 6 carbon atoms, or an alkylene of 2 to 6 carbon atoms interrupted by pyrrolidinediyl, piperidinediyl or piperazinediyl.

The heterocyclic moiety of the "bivalent heterocyclic group" is preferably pyrrolidinediyl, piperidinediyl or piperazinediyl.

The halo(lower)alkyl group which may represent W, X and/or Y may be the above defined lower alkyl substituted by one or more halogen atoms as defined above in association with W, X and Y, more preferably a mono- or di- or trihaloalkyl of 1 to 4 carbon atoms, and most preferably trifluoromethyl.

The acid-addition salt of dihydropyridine derivatives of the invention may be for example, a salt derived from an inorganic acid, for example a hydrochloride or sulphate, or a salt derived from an organic acid, for example an oxalate, lactate, succinate, tartrate, maleate, fumarate, acetate, salicylate, citrate, benzoate, beta-naphthoate, adipate, methanesulphonate, benzenesulphonate or p-toluenesulphonate.

The dihydropyridine derivatives of the invention may be produced by any chemical proccess known to be useful for manufacture of chemically analogous compounds.

One preferred process for manufacturing the dihydropyridine derivatives of this invention comprises the reaction of an amine of the formula:

NH₂R

wherein R has the meanings stated above or a salt thereof, with an epoxide or an alcohol derivative of the respective formulae: 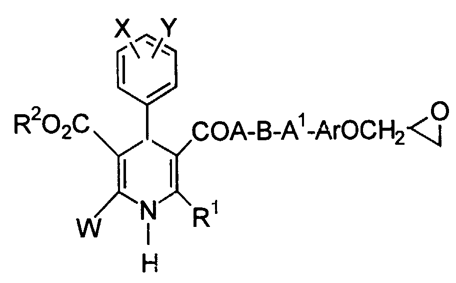 and 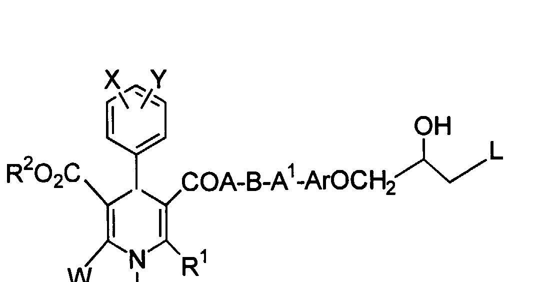 wherein W, R¹, R², X, Y, A, B, A¹, and Ar have the meanings stated above, and L stands for a suitable leaving group (reactive functional group), for example, methanesulphonyloxy, benzenesulphonyloxy, p-toluenesulphonyloxy, m-nitrobenzenesulphonyloxy, p-nitrobenzenesulphonyloxy, chloro, bromo or iodo group.

A second preferred process for manufacturing the dihydropyridine derivatives of this invention comprises the reaction of a dihydropyridine derivative of the formula: 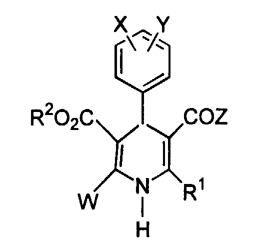 wherein W, R¹, R², X and Y have the meanings stated above, and Z stands for a suitable leaving group (reactive functional group), for example, a hydroxy group or a halogen group, with a compound of the formula:

H-A-B-A¹-ArOCH₂CHOHCH₂NHR

A third preferred process for manufacturing the dihydropyridine derivatives of this invention comprises the reaction of a dihydropyridine derivative of the formula: 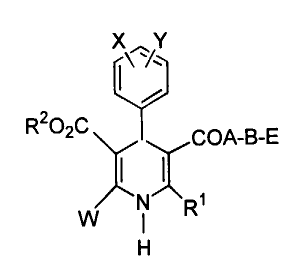 wherein W, R¹, R², X and Y have the meanings stated above, and E stands for a suitable leaving group (reactive functional group), for example a hydroxy group or a halogen group, with a compound of the formula: 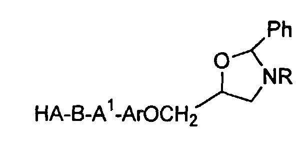 wherein R, A, B, A¹ and Ar have the meanings stated above and wherein Ph represents a phenyl group, and successsively a removal of N,O-benzylidene acetal by hydrolysis using an acid such as NH₄Cl aq., dil.HCl or silica gel.

A fourth preferred process for manufacturing the dihydropyridine derivatives of this invention comprises the reaction of a dihydropyridine derivative of the formula: 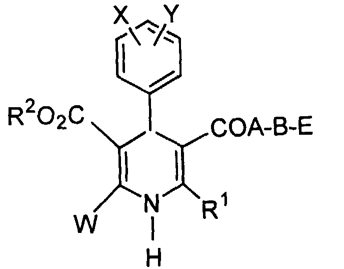 wherein W, R¹, R², X, y, A and B have the meanings stated above, and E stands for a suitable leaving group (reactive functional group), for example a halogen group or a p-toluenesulphonyloxy group, with a compound of the formula:

H₂NArOCH₂CHOHCH₂NHR

wherein R and Ar have the meanings defined above.

Another suitable process for manufacturing the dihydropyridine derivatives of this invention is the reduction of an aminoketone of the formula: 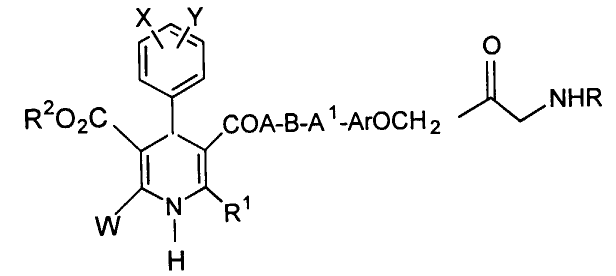 wherein W, R¹, R², R, A, B, A¹, Ar, X and Y have the meanings stated above, with a reducing agent such as sodium borohydride in alcohol.

The dihydropyridine reaction product of this invention can be separated and isolated from the reaction mixture and purified by methods commonly used for such purpose, for instance, extraction with a suitable solvent, chromatography, precipitation, recrystallization etc.

The dihydropyridines of this invention includes at least two pairs of optical isomers due to the presence of an asymmetric carbon atom of the fourth position of the 1, 4-dihydropyridine nucleus and an asymmetric carbon atom in the 3-aryloxy-2-hydroxypropylamine moiety and thus can exist as each optical isomer or a mixture thereof. A racemic mixture of the optical isomers can be resolved into each optical isomer by a conventional method for optical resolution, such as a chemical resolution of the salts of the diastereomer with a conventional optically active acid (e.g. tartaric acid or camphor sulfonic acid, etc).

The compounds of this invention and their pharmaceutically acceptable salts possess strong and long lasting vasodilating and anti-hypertensive activities and are useful for therapeutical treatment in cardiovascular disorders and hypertension such as coronary insufficiency, angina pectoris or myocardinal infarction, and hypertension.

The favourable pharmacological activities of the compound according to this invention are structurally characterized by the radicals W, R¹, R², R, A, B, A¹, Ar, X, Y and the substitution pattern of the substituents on the phenyl ring at the fourth position of the dihydropyridine nucleus.

In a more specific embodiment of the present invention, the radical W may be cyano, trifluoromethyl, difluoromethyl, monofluoromethyl, 2,2,2-trifluoroethyl, trichloromethyl, dichloromethyl, monochloromethyl, 2,2,2-trichloroethyl and preferably is selected from cyano and trifluoromethyl; the radicals R, R¹ and R² are independently selected from the group consisting of methyl, ethyl, propyl, isopropyl, butyl, isobutyl, amyl, isoamyl, hexyl, isohexyl and more preferably is selected from methyl, ethyl, propyl, isopropyl, butyl, isobutyl; the radical -A- is selected from -O-, -NH- and more preferably -O-; the radical -A¹- is the group comprising direct link, -O-, -NH-, -N(CH₂)ₚCH₃-, -NHCO-, -CONH-, -NHCO(CH₂)ₘ-, -CONH(CH₂)ₘ-, -N(CH₂)ₚCH₃CO-, -CON(CH₂)ₚCH₃-, -N(CH₂)ₚCH₃CO(CH₂)ₘ-, -CON(CH₂)ₚCH₃(CH₂)ₘ-, -o-(CH₂)ₘ-O-, -NH-(CH₂)ₘ-, -N{(CH₂)ₚCH₃}-(CH₂)ₘ₋, 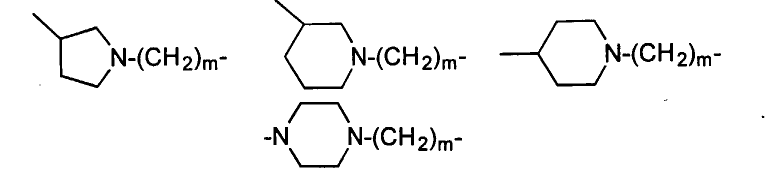 wherein m is 2,3,4 or 5, and wherein p is 0,1,2,3,4 or 5, and more preferably a direct link, -O-, -NH-; the radical B is selected from the group consisting of a direct link, methylene, ethylene, propylene, trimethylene, tetramethylene, pentamethylene, hexamethylene, heptamethylene, octamethylene, and more preferably is methylene, ethylene, trimethylene; the radical Ar is selected from 1,4-phenylene, 1,3-phenylene, 1,2-phenylene, 2-methoxy,1,4-phenylene, 3-methoxy-1,4-phenylene, 2-methoxy-1,3-phenylene, 4-methoxy-1,3-phenylene, 5-methoxy-1,3-phenylene, 6-methoxy-1,3-phenylene, 3-methoxy-1,2-phenylene, 4-methoxy-1,2-phenylene, 5-methoxy-1,2-phenylene, 6-methoxy-1,2-phenylene; the radicals X and Y are independently and preferably selected from the group consisting of hydrogen, nitro, chloro, fluoro and more preferably hydrogen, nitro, chloro. The preferable substitution pattern of the substituents on the phenyl ring of the fourth position on the dihydropyridine nucleus is selected from 1,2,3- 1,2,4-, 1,2,5- 1,2,6-, 1,3,4-, 1,3,5-, 1,2- 1,3- and 1,4-.

For therapeutical purposes, the dihydropyridine derivatives of this invention may be administered in a daily dosage of 0.1 to 500 mg. preferably 1 to 250 mg.

The pharmaceutical compositions of this invention may comprise, as an active ingredient, the dihydropyridine compound or a pharmaceutically acceptable salt thereof in an amount of about 0.01 mg to about 500 mg., preferably about 0.1 mg to about 250 mg per dosage for oral and parenteral use.

One skilled in the art will recognize that the amount of the active ingredient in the dosage unit form may be determined by considering the activity of the ingredient as well as the size of the host human being. The active ingredient may usually be formulated in a solid form such as tablet, granule, powder, capsule, troche, lozenge or suppository, or a suspension of solution form such as syrup, injection, emulsion, lemonade, etc. and the like. A pharmaceutical carrier or diluent includes solid or liquid non-toxic pharmaceutically acceptable substances. Examples of solid or liquid carriers or diluents are lactose, magnesium stearate, terra alba, sucrose, corn starch, talc, stearic acid, gelatin, agar, pectin, acacia, peanut oil, olive oil, or sesame oil, cacao butter, ethyleneglycol or the other conventional ones. Similarly, the carrier or diluent may include a time delay material such as glyceryl monostearate, glyceryl distearate, a wax and the like.

For the purpose of showing the utility of the compound of this invention, the pharmacological test results of a representative compound will be shown as follows:

### 1) Hypotensive effect

### Test method

Three Wistar rats were used per group. Each animal was placed in a cage sized to the body after cannulation of catheters into the femoral artery and vein under ether anesthesia. Blood pressure was measured in the femoral artery by means of a pressure transducer and recorded as electrically integrated values of mean arterial pressure. Heart rate was counted from instant arterial pressure pulses by running the record paper at a faster speed periodically.

The test compound was administered intravenously through a catheter cannulated in the femoral vein more than 2 hours later the completion of the operation.

### Test compound

Dihydropyridine A (The product of Example 2)

### Test results

| | | |
|---|---|---|
| Test compound | dose(mg/kg) | Maximum change of blood pressure |
| Dihydropyridine A | 1.0 | -39(%) |

### (2) Ca channel binding assay

### Test methods

### (a) Crude vascular microsomal membrane preparation

Porcine thoracic aorta was obtained from a slaughterhouse. The fat and connective tissues were removed and the vascular tissue was frozen at -80°C.

The frozen tissue was thawed in the buffer (0.25M sucrose, 10mM MOPS, pH7.4) and homogenized in the same buffer by polytoron (Kinematica). After filtration of the homogenate through gauzes, the filtrated homogenate was homogenized by teflon glass homogenizer. The homogenate was centrifuged (1,000g x 10 min). The supernatant was centrifuged (10,000gx10 min) twice. The supernatant was centrifuted (100,000g x 60 min) to yield pellets. The pellets were resuspended in buffer (50mM Tris-HCl, pH 7.4), which were referred to as crude microsomal membrane fractions. The obtained suspensions were stored at -80°C until use.

### (b) [³H]PN200-110 binding to preparative membrane

Frozen crude microsomal fractions were thawed. [³H] Pn200-110 (0.1nM) was incubated with 50 µl of the membrane preparation at 37°C for 90 minutes in a final volume of 200 µl. At the end of the incubation period, reaction mixture was quickly filtrated over a Whatman GF/C glass filter under aspiration. The filters were than washed 5 times with 3 ml of the buffer (50mM Tris-HCl, pH7.4). The radioactivity was counted in 6 ml of Clear-sol I in Packard scintillation counter (Packard TRl-CARB 4530).

### Test compound

Dihydropyridine A

### Test result

| | |
|---|---|
| Test compound | Kᵢ(M) |
| Dihydropyridine A | 2.97 x 10⁻⁹ |

### (3) β receptor binding

### Test methods

### (a) Crude cardiac microsomal membrane preparation

SD strain rats were sacrificed by decapitation. The heart was removed and homogenized in buffer (0.25M sucrose, 10mM MOPS, pH7.4) by using Polytoron (Kinematica). The homogenate was homogenized by teflon glass homogenizer. The homogenate was centrifuged (1,000g x 10 min) to remove tissue clumps and the supernatant was centrifuged (10,000g x 10 min) twice. The supernatant was centrifuged (100,000g x 60 min) to yield pellets. The pellets were resuspended in buffer (50mM Tris-HCl, pH7.4), which were referred to as crude microsomal membrane fractions. The obtained suspensions were stored at -80°C until use.

### (b) ³H-Dihydroalprenolol (DHA) binding to preparative membrane

Frozen crude microsomal membrane fractions were thawed. [³H) DHA (1nM) was incubated with 50 µl of the membrane preparation at 25°C for 30 minutes in a final volume of 200 µl. At the end of the incubation period, reaction mixture was quickly filtrated over a Whatman GF/C glass filter under aspiration. The filters were washed 5 times with 3ml of the buffer (50mM Tris-HCl, pH7.4). The radioactivity was counted in 6ml of Clear-sol I in Packard scintillation counter (Packard TRI-CARB 4530).

### Test compound

Dihydropyridine A

### Test result

| | |
|---|---|
| Test compound | Kᵢ(M) |
| Dihydropyridine A | 5.07 x 10⁻⁷ |

The invention is illustrated but not limited by the following Examples:-

### Example 1

Preparation of p-(2-hydroxy-3-isopropylaminopropoxy)benzyl 1,4-dihydro-3-methoxycarbonyl-6-methyl-4-m-nitrophenyl-2-trifluoromethylpyridine-5-carboxylate.

A mixture of p-(2,3-epoxypropoxy)benzyl 1,4-dihydro-3-methoxycarbonyl-6-methyl-4-m-nitrophenyl-2-trifluoromethylpyridine-5-carboxylate (650 mg) and isopropylamine (2.0 ml) in ethanol (8.0 ml) was refluxed for 2h. The reaction mixture was concentrated under reduced pressure. The residue was chromatographed using a silica gel column (chloroform/methanol=10/1 v/v). p-(2-Hydroxy-3-isopropylaminopropoxy)benzyl 1,4-dihydro-3-methoxycarbonyl-6-methyl-4-m-nitrophenyl-2-trifluoromethylpyridine-5-carboxylate was obtained as a yellow stiff foam (620 mg).
IR (KBr) 3342, 2964, 1706, 1530, 1514, 1349, 1218, 1176, 1075, 827, 740 cm⁻¹;
¹H-NMR (CDCl₃-TMS) δ 8.03(d, 1H), 7.99(d, 1H), 7.53(d, 1H), 7.37(t, 1H), 7.12(d, 2H), 6.83(d, 2H), 6.33(s,1H), 5.12(s, 1H), 5.08(d, 1H), 4.93(d, 1H), 4.05-4.00(m, 1H), 3.98(d, 2H), 3.68(s, 3H), 2.90(dd, 1H), 2.85(quint., 1H), 2.75(dd, 1H), 2.43(s, 3H), 1.11(d, 6H);
mass spectrum, m/e(FD) 608 (M⁺+1).

### Example 2

Preparation of 2-{p-(2-hydroxy-3-isopropylaminopropoxy)phenyl}ethyl 1,4-dihydro-3-methoxycarbonyl-6-methyl-4-m-nitrophenyl-2-trifluoromethylpyridine-5-carboxylate.

The procedure described in Example 1 was followed using 2-{p-(2,3-epoxypropoxy)phenyl}ethyl 1,4-dihydro-3-methoxycarbonyl-6-methyl-4-m-nitrophenyl-2-trifluoromethylpyridine-5-carboxylate (540 mg) and isopropylamine (2.0 ml) as starting materials. 2-{p-(2-Hydroxy-3-isopropylaminopropoxy)phenyl}ethyl 1,4-dihydro-3-methoxycarbonyl-6-methyl-4-m-nitrophenyl-2-trifluoromethylpyridine-5-carboxylate was obtained as a yellow stiff foam (560mg).
IR (KBr) 3341, 2965, 1706, 1349, 1280, 1221, 1179, 1085, 826 cm⁻¹;
¹H-NMR(CDCl₃-TMS) δ 8.05-8.03(m, 2H), 7.51(d, 1H), 7.39(t, 1H), 7.04(d, 2H), 6.80(d, 2H), 6.47(s, 1H), 5.11(s, 1H), 4.27(t, 2H), 4.06-4.17(m, 1H), 3.95(dd, 2H), 3.72(s, 3H), 2.92-2.72(m, 5H),m 2.36(s, 3H), 1.12(d, 6H);
mass spectrum, m/e(FD) 622 (M⁺+1).

### Example 3

Preparation of 2-{p-(2-hydroxy-3-isopropylaminopropoxy)phenyl}ethyl 3-ethoxycarbonyl-1,4-dihydro-6-methyl-4-m-nitrophenyl-2-trifluoromethylpyridine-5-carboxylate.

The procedure described in Example 1 was followed using 2-{p-(2,3-epoxypropoxy)phenyl}ethyl 3-ethoxycarbonyl-1,4-dihydro-6-methyl-4-m-nitrophenyl-2- trifluoromethylpyridine-5-carboxylate (350 mg) and isopropylamine (1.0 ml) as starting materials. 2-{p-(2-Hydroxy-3-isopropylaminopropoxy)phenyl}ethyl 3-ethoxycarbonyl-1,4-dihydro-6-methyl-4-m-nitrophenyl-2- trifluoromethylpyridine-5-carboxylate was obtained as a yellow stiff foam (360 mg).
IR (KBr) 3342, 2966, 1704, 1626, 1530, 1513, 1349, 1221, 1178, 1082, 824, 778 cm⁻¹;
¹H-NMR (CDCl₃-TMS) δ 8.06-8.04(m, 2H), 7.52(d, 1H), 7.39(t, 1H), 7.03(d, 2H), 6.80(d, 2H), 6.38(s, 1H), 5.11(s, 1H), 4.26(t, 2H), 4.20-4.13(m, 2H), 4.10-4.04(m, 1H), 3.95(dd, 2H), 2.94-2.74(m, 5H), 2.37(s, 3H), 1.24(t, 3H), 1.13(d, 6H);
mass spectrum, m/e(FD) 636 (M⁺+1).

### Example 4

Preparation of 2-{p-(2-hydroxy-3-tert-butylaminopropoxy)phenyl}ethyl 3-ethoxycarbonyl-1,4-dihydro-6-methyl-4-m-nitrophenyl-2- trifluoromethylpyridine-5-carboxylate.

The procedure described in Example 1 was followed using 2-{p-(2,3-epoxypropoxy)phenyl}ethyl 3-ethoxycarbonyl-1,4-dihydro-6-methyl-4-m-nirophenyl-2-trifluoromethylpyridine-5-carboxylate (340 mg) and tert-butylamine (1.0 ml) as starting materials. 2-{p-(2-Hydroxy-3-tert-butylaminopropoxy)phenyl}ethyl 3-ethoxycarbonyl-1,4-dihydro-6-methyl-4-m-nitrophenyl-2-trifluoromethylpyridine-5-carboxylate was obtained as a yellow stiff foam (330 mg).
IR (KBr) 3342, 2971, 1702, 1625, 1530, 1513, 1349, 1220, 1178, 1097, 826, 780 cm⁻¹;
¹H-NMR (CDCl₃-TMS) δ 8.05-8.03(M, 2H), 7.53(d, 1H), 7.40(t, 1H), 7.02(d, 2H), 6.79(d, 2H), 6.29(s, 1H), 5.11(s, 1H), 4.33(br.s, 1H), 4.26(t, 2H), 4.20-4.14(m, 2H), 4.03(dd, 1H), 3.96(dd, 1H) 3.13(br.s, 1H), 2.94(br.s, 1H), 2.83(t, 2H), 2.36(s, 3H), 1.35(s, 9H), 1.24(t, 3H):
mass spectrum, m/e(FD) 650 (M⁺+1).

### Example 5

Preparation of 3-{p-(2-hydroxy-3-isopropylaminopropoxy)phenyl}propyl 1,4-dihydro-3-methoxycarbonyl-6-methyl-4-m-nitrophenyl-2-trifluoromethylpyridine-5-carboxylate monohydrochloride.

A mixture of 3-{p-(2,3-epoxypropoxy)phenyl}propyl 1,4-dihydro-3-methoxycarbonyl-6-methyl-4-m-nitrophenyl-2-trifluoromethylpyridine-5-carboxylate (540 mg) and isopropylamine (2.0 ml) in ethanol (8.0 ml) was refluxed for 2h. The reaction mixture was concentrated under reduced pressure. The residue was chromatographed using a silica gel column (chloroform/methanol=10/1 v/v). 3-{p-(2-Hydroxy-3-isopropylaminopropoxy)phenyl}propyl 1,4-dihydro-3-methoxycarbonyl-6-methyl-4-m-nitrophenyl-2-trifluoromethylpyridine-5-carboxylate was obtained as a yellow oil.

To a solution of 3-{p-(2-hydroxy-3-isopropylaminopropoxy)phenyl}propyl 1,4-dihydro-3-methoxycarbonyl-6-methyl-4-m-nitrophenyl-2-trifluoromethylpyridine-5-carboxylate in methanol (3.0 ml) was added 2N methanol solution of hydrogen chloride (1.0 ml). The reaction mixture was stirred for 1 h at room temperature. Methanol was evaporated under reduced pressure. 3-{p-(2-Hydroxy-3-isopropylaminopropoxy)phenyl}propyl 1,4-dihydro-3-methoxycarbonyl-6-methyl-4-m-nitrophenyl-2-trifluoromethylpyridine-5-carboxylate monohydrochloride was obtained as a yellow stiff foam (530 mg).
IR (KBr) 3350, 2953, 1708, 1627, 1530, 1512, 1350, 1281, 1232, 1079, 1059, 884, 852 cm⁻¹;
¹H-NMR (CDCl₃-TMS) δ 8.12(t. 1H), 8.08-8.06(m, 1H), 7.64(d, 1H), 7.44(t, 1H), 6.99(d, 2H), 6.81(d, 2H), 6.39(s, 1H), 5.18(s, 1H), 4.11-4.01(m, 3H), 4.00-3.92(m, 2H), 3.73(s, 3H), 2.96-2.90(m, 2H), 2.80-2.72(m, 1H) 2.51(t, 2H), 2.44(s, 3H), 1.88(p, 2H), 1.15(d, 6H);
mass spectrum, m/e(FD) 636 (M⁺+1).

### Example 6

Preparation of 3-{p-(2-hydroxy-3-tert-butylaminopropoxy)phenyl}propyl 1,4-dihydro-3-methoxycarbonyl-6-methyl-4-m-nitrophenyl-2-trifluoromethylpyridine-5-carboxylate monohydrochloride.

The procedure described in Example 5 was followed using 3-{p-(2,3-epoxypropoxy)phenyl}propyl 1,4-dihydro-3-methoxycarbonyl-6-methyl-4-m-nitrophenyl-2-trifluoromethylpyridine-5-carboxylate (540 mg) and tert-butylamine (2.0 ml) as starting materials. 3-{p-(2-Hydroxy-3-tertbutyl aminopropoxy)phenyl}propyl 1,4-dihydro-3-methoxycarbonyl-6-methyl-4-m-nitrophenyl-2-trifluoromethylpyridine-5- carboxylate monohydrochloride was obtained as a yellow stiff foam (530 mg).
IR (KBr) 3353, 2955, 2359, 1708, 1626, 1530, 1513, 1439, 1350, 1230, 1098, 851, 580 cm⁻¹;
¹H-NMR (CDCl₃-TMS) δ 8.18(s, 1H), 8.03(d, 1H), 7.63(d, 1H), 7.40(t, 1H), 6.92(d, 2H), 6.75(d, 2H), 6.58(s, 1H), 5.15(s, 1H), 4.55(br.s, 1H), 4.10-3.90(m, 4H), 3.71(s, 3H), 3.25-3.10(m, 2H), 2.50-2.40(m, 2H, 2.41(s,3H), 1.90-1.80(m, 2H), 1.39(s, 9H);
mass spectrum, m/e(FD) 650 (m⁺+H).

### Example 7

Preparation of 3-{p-(2-hydroxy-3-isopropylaminopropoxy)phenyl}propyl 3-ethoxycarbonyl-1,4-dihydro-6-methyl-4-m-nitrophenyl-2-trifluoromethylpyridine-5-carboxylate monohydrochloride.

The procedure described in Example 5 was followed using 3-{p-(2,3-epoxypropoxy)phenyl}propyl 3-ethoxycarbonyl-1,4-dihydro-6-methyl-4-m-nitrophenyl-2- trifluoromethylpyridine-5-carboxylate (540 mg) and isopropylamine (2.0 ml) as starting materials. 3-{p-(2-Hydroxy-3-isopropylaminopropoxy)phenyl}propyl 3-ethoxycarbonyl-1,4-dihydro-6-methyl-4-m-nitrophenyl-2- trifluoromethylpyridine-5-carboxylate monohydrochloride was obtained as a yellow stiff foam (540 mg).
IR (KBr) 3341, 2983, 1702, 1625;, 1530, 1513, 1349, 1279, 1220, 1179, 1097, 827, 740 cm⁻¹;
¹H-NHMR (CDCl₃-TMS) δ 3.13(t, 1H), 8.06(dd, 1H), 7.64(d, 1H), 7.44(t, 1H), 6.96(d, 2H), 6.78(d, 2H), 6.39(s, 1H), 5.17(s, 1H), 4.63(br.s, 1H), 4.20-4.15(m, 2H), 4.08-3.97(m, 4H), 3.47(br.s, 1H), 3.30(Br.s, 1H) 3.18(br.s, 1H) 2.49(t, 2H), 2.43(s, 3H), 1.89-1.83(m, 2H), 1.49(t, 6H), 1.24(t, 3H);
mass spectrum, m/e(FD) 650 (M⁺+1).

### Example 8

Preparation of 3-{p-(2-hydroxy-3-tert-butylaminopropoxy)phenyl}propyl 3-ethoxycarbonyl-1,4-dihydro-6-methyl-4-m-nitrophenyl-2-trifluoromethylpyridine-5-carboxylate monohydrochloride.

The procedure described in Example 5 was followed using 3-{p-(2,3-epoxypropoxy)phenyl}propyl 3-ethoxycarbonyl-1,4-dihydro-6-methyl-4-m-ntrophenyl-2-trifluoromethylpyridine-5-carboxylate (560 mg) and tert-butylamine (2.0 ml) as starting materials 3-{p-(2-Hydroxy-3-tert-butylaminopropoxy)phenyl}propyl 3-ethoxycarbonyl-1,4-dihydro-6-methyl-4-m-nitrophenyl-2-trifluoromethylpyridine-5-carboxylate monohydrochloride was obtained as a yellow stiff foam (540 mg).
IR (KBr) 3333, 2981, 2784, 1704, 1682, 1530, 1512, 1382, 1349, 1219, 1178, 1097, 827, 781cm⁻¹;
¹H-NMR (CDCl₃-TMS) δ 8.13(s, 1H), 8.06(d, 1H), 7.64(d, 1H), 7.44(t, 1H), 6.97(d, 2H, 6.80(d, 2H), 6.39(s, 1H), 5.18(s, 1H), 4.62(br.s, 1H), 4.20-3.90(m, 6H), 3.31(br.s, 1H), 3.11(br.s, 1H), 2.50(t, 2H), 1.87(p, 2H), 1.50(s, 9H), 1.24(t, 3H);
mass spectrum, m/e(FD) 664 (M⁺+1).

### Example 9

Preparation of 2-{4-(2-hydroxy-3-isopropylaminopropoxy)-3-methoxyphenyl}ethyl 1,4-dihydro-3-methoxycarbonyl-6-methyl-4-m-nitrophenyl-2-trifluoromethylpyridine-5-carboxylate.

The procedure described in Example 1 was followed using 2-{4-(2,3-epoxypropoxy)-3-methoxyphenyl}ethyl 1,4-dihydro-3-methoxycarbonyl-6-methyl-4-m-nitrophenyl -2-trifluoromethylpyridine-5-carboxylate (690 mg) and isopropylamine (1.0 ml) as starting materials. 2-{4-(2-Hydroxy-3-isopropylaminopropoxy)-3-methoxyphenyl}ethyl 1,4-dihydro-3-methoxycarbonyl-6-methyl-4-m-nitrophenyl-2-trifluoromethylpyridine-5-carboxylate was obtained as a yellow stiff foam (590 mg).
IR (KBr) 3316, 2966, 1705, 1531, 1644, 1396, 1349, 1275, 1219, 1142, 1080, 1020, 780 cm⁻¹;
¹H-NMR (CDCl₃-TMS) δ 8.06-8.04(m, 2H), 7.51(d, 1H), 7.39(t, 1H), 6.81(d, 1H), 6.67(s, 1H), 6.66(d, 1H), 6.34(s, 1H), 5.11(s, 1H), 4.29(t, 2H), 4.17-4.16(m, 1H), 4.06-3.99(m, 2H), 3.80(s, 3H), 3.71(s, 3H), 3.03-2.97(m, 2H), 2.90-2.83(m, 3H), 2.36(s, 3H), 1.19(d, 6H);
mass spectrum, m/e(FD) 652 (M⁺+1).

### Example 10

Preparation of 2-{4-(2-hydroxy-3-isopropylaminopropoxy)-3-methoxyphenyl}ethyl 3-ethoxycarbonyl-1,4-dihydro-6-methyl-4-m-nitrophenyl-2- trifluoromethylpyridine-5-carboxylate.

The procedure described in Example 1 was followed using 2-{4-(2,3-epoxypropoxy)-3-methoxyphenyl}ethyl 3-ethoxycarbonyl-1,4-dihydro-6-methyl-4-m-nitrophenyl-2-trifluoromethylpyridine-5-carboxylate (740 mg) and isopropylamine (1.0 ml) as starting materials. 2-{4-(2-Hydroxy-3-isopropylaminopropoxy)-3-methoxyphenyl}ethyl 3-ethoxycarbonyl-1,4-dihydro-6-methyl-4-m-nitrophenyl-2-trifluoromethylpyridine-5- carboxylate was obtained as a yellow stiff foam (680 mg).
IR (KBr) 3316, 2966, 1705, 1531, 1644, 1396, 1349, 1275, 1219, 1142, 1080, 1020, 780 cm⁻¹ ;
¹H-NMR (CDCl₃-TMS) δ 8.06-8.04(m, 2H), 7.52(d, 1H), 7.39(t, 1H), 6.81(d, 1H), 6.67(s, 1H), 6.66(d, 1H), 6.31(s, 1H), 5.12(s, 1H), 4.28(t, 2H), 4.30-4.10(m, 3H), 4.05-3.98(m, 2H), 3.81(s, 3H), 2.98-2.92(m, 2H), 2.86-2.83(m, 3H), 2.37(s, 3H), 1.23(t, 3H), 1.16(d, 6H);
mass spectrum, m/e(FD) 666 (M⁺+1).

### Example 11

Preparation of 2-{o-(2-hydroxy-3-isopropylaminopropoxy)phenyl}ethyl 1,4-dihydro-3-methoxycarbonyl-6-methyl-4-m-nitrophenyl-2-trifluoromethylpyridine-5-carboxylate.

The procedure described in Example 1 was followed using 2-{o-(2,3-epoxypropoxy)phenyl}ethyl 1,4-dihydro-3-methoxycarbonyl-6-methyl-4-m-nitrophenyl-2-trifluoromethylpyridine-5-carboxylate (740 mg) and isopropylamine (1.0 ml) as starting materials. 2-{o-(2-Hydroxy-3-isopropylaminopropoxy)phenyl}ethyl 1,4-dihydro-3-methoxycarbonyl-6-methyl-4-m-nitrophenyl-2-trifluoromethylpyridine-5-carboxylate was obtained as a yellow stiff foam (600 mg).
IR (KBr) 3350, 2965, 1705, 1627, 1350, 1496, 1350, 1223, 1182, 1083, 753 cm⁻¹;
¹H-NMR (CDCl₃-TMS) δ 8.05-8.04(m, 2H), 7.51(d, 1H), 7.40(t, 1H), 7.18(t, 1H), 7.06(d, 1H), 6.85(t, 1H), 6.83(d, 1H), 6.29(s, 1H), 5.12(s, 1H), 4.35-4.25(m, 2H), 4.15-4.14(m, 1H), 3.99-3.95(m, 2H), 3.72(s, 3H), 2.97-2.88(m, 3H), 2.82(dd, 1H), 2.39(s, 3H), 1.16(d, 6H);
mass spectrum, m/e (FD) 623 (M⁺+1).

### Example 12

Preparation of 2-{o-(2-hydroxy-3-isopropylaminopropoxy)phenyl}ethyl 3-ethoxycarbonyl-1,4-dihydro-6-methyl-4-m-nitrophenyl-2-trifluoromethylpyridine-5-carboxylate.

The procedure described in Example 1 was followed using 2-{o-(2,3-epoxypropoxy)phenyl}ethyl 3-ethoxycarbonyl-1,4-dihydro-6-methyl-4-m-nitrophenyl-2-trifluoromethylpyridine-5-carboxylate (720 mg) and isopropylamine (1.0 ml) as starting materials. 2-{o-(2-Hydroxy-3-isopropylaminopropoxy)phenyl}ethyl 3-ethoxycarbonyl-1,4-dihydro-6-methyl-4-m-nitrophenyl-2-trifluoromethylpyridine-5-carboxylate was obtained as a yellow stiff foam (650 mg).
IR (KBr) 3345, 3110, 2968, 2871, 1704, 1624, 1531, 1496, 1350, 1222, 1181, 1124, 1081, 751 cm⁻¹;
¹H-NMR (CDCl₃-TMS) δ? 8.06(s, 1H), 8.05(d, 1H), 7.53(d, 1H), 7.40(t, 1H), 7.18(t, 1H), 7.06(d, 1H), 6.85(t, 1H), 6.83(d, 1H), 6.27(s, 1H), 5.12(s, 1H), 4.35-4.09(m, 5H), 4.00-3.94(m, 2H), 2.93-2.86(m, 4H), 2.78(dd, 1H), 2.39(s, 3H), 1.23(t, 3H), 1.12(d, 6H);
mass spectrum, m/e(FD) 636 (M⁺+1).

### Example 13

Preparation of 2-{p-(2-hydroxy-3-isopropylaminopropoxy)phenyl}ethyl 2-cyano-1,4-dihydro-3-methoxycarbonyl-6-methyl-4-m-nitrophenylpyridine-5-carboxylate.

The procedure described in Example 1 was followed using 2-{p-(2,3-epoxypropoxy)phenyl}ethyl 2-cyano-1,4-dihydro-3-methoxycarbonyl-6-methyl-4-m-nitrophenylpyridine-5-carboxylate (500 mg) and isopropylamine (0.5 ml) as starting materials. 2-{p-(2-Hydroxy-3-isopropylaminopropoxy)phenyl}ethyl 2-cyano-1,4-dihydro-3-methoxycarbonyl-6-methyl-4-m- nitrophenylpyridine-5-carboxylate was obtained as an orange stiff foam (400 mg).
IR (KBr) 3299, 3091, 2962, 2236, 1705, 1644, 1614, 1583, 1529, 1512, 1470, 1435, 1384, 1349, 1298, 1272, 1247, 1216, 1178, 1098, 1029, 1000, 924, 901, 826, 782, 753, 704 cm⁻¹;
¹H-NMR (CDCl₃-TMS) δ 8.05(d, 1H), 8.01(s, 1H), 7.51(d, 1H), 7.39(dd, 1H), 7.01(d, 2H), 6.79(d, 2H), 5.14(s, 1H), 4.27(dd, 2H), 4.15(m, 1H), 3.97(dd, 2H), 3.78(s, 3H), 2.99(dd, 2H), 2.9-2.8(m, 3H), 2.5(br.s, 3H), 2.30(s, 3H), 1.19 and 1.18(2s, 6H).
mass spectrum, m/e(FD) 579 (M⁺+1).

### Example 14

Preparation of 3-{p-(2-Hydroxy-3-isopropylaminopropoxy)phenyl}propyl 2-cyano-1,4-dihydro-3-methoxycarbonyl-6-methyl-4-m-nitrophenylpyridine-5-carboxylate.

The procedure described in Example 1 was followed using 3-{p-(2,3-epoxypropoxy)phenyl}propyl 2-cyano-1,4-dihydro-3-methoxycarbonyl-6-methyl-4-m-nitrophenylpyridine-5-carboxylate (500 mg) and isopropylamine (0.5 ml) as starting materials. 3-{p-(2-Hydroxy-3-isopropylaminopropoxy)phenyl}propyl 2-cyano-1,4-dihydro-3-methoxycarbonyl-6-methyl-4-m-nitrophenylpyridine-5-carboxylate was obtained as an orange stiff foam (170 mg).
IR (KBr) 3239, 3078, 2958, 2237, 1705, 1643, 1614, 1584, 1530, 1512, 1471, 1436, 1385, 1349, 1298, 1272, 1216, 1099, 1031, 954, 899, 826, 782, 750, 703 cm⁻¹;
¹H-NMR (CDCl₃-TMS) δ 8.096, 8.092(2d, 1H), 8.05(dd, 1H), 7.53(d, 1H), 7.42(dd, 1H), 6.974 and 6.967(2d, 2H), 6.785 and 6.781(2d, 2H), 5.158 and 5.153(2s, 1H), 4.17(m, 1H), 4.1-3.9(m, 4H), 3.78(s, 3H), 3.0-2.8(m, 3H), 2.51(dd, 2H), 2.362 and 2.357(2s, 3H), 1.88(m, 2H), 1.18(d, 6H);
mass spectrum, m/e(FD) 593 (M⁺+1).

### Example 15

Preparation of 2-{o-(2-Hydroxy-3-isopropylaminopropoxy)phenyl}ethyl 2-cyano-1,4-dihydro-3-methoxycarbonyl-6-methyl-4-m-nitrophenylpyridine-5-carboxylate.

The procedure described in Example 1 was followed using 2-{o-(2,3-epoxypropoxy)phenyl}ethyl 2-cyano-1,4-dihydro-3-methoxycarbonyl-6-methyl-4-m-nitrophenylpyridine-5-carboxylate (530 mg) and isopropylamine (0.5 ml) as starting materials. 2-{o-(2-Hydroxy-3-isopropylaminopropoxy)phenyl}ethyl 2-cyano-1,4-dihydro-3-methoxycarbonyl-6-methyl-4-m-nitrophenylpyridine-5-carboxylate was obtained as an orange stiff foam (200 mg).
IR (KBr) 3434, 3079, 2964, 2236, 1704, 1627, 1558, 1530, 1496, 1472, 1455, 1436, 1385, 1349, 1310, 1298, 1272, 1247, 1217, 1120, 1098, 1031, 1001, 953, 923, 753, 705 cm⁻¹;
¹H-NMR (CDCl₃-TMS) δ 8.068, 8.065(2s, 1H), 8.043 and 8.026(2d, 1H), 7.546 and 7.517(2d, 1H), 7.401 and 7.376(2dd, 1H), 7.19-7.17(m, 1H), 7.093 and 7.055(2d, 1H), 6.870 and 6.855(2dd, 1H), 6.813(d, 1H), 5.176 and 5.159(2s, 1H), 4.45-3.83(m, 6H), 3.771 and 3.765(2s, 3H), 3.05-2.75(m, 4H), 2.463-2.405(2s, 3H), 1.171 and 1.158(2d, 3H), 1.154 and 1.141(2s, 3H);
mass spectrum, m/e(FD 579 (M⁺+1).

### Example 16

Preparation of p-(2-hydroxy-3-isopropylaminopropoxy)benzyl 2-cyano-1,4-dihydro-3-methoxycarbonyl-6-methyl-4-m-nitrophenylpyridine-5-carboxylate.

The procedure described in Example 1 was followed using p-(2,3-epoxypropoxy)benzyl 2-cyano-1,4-dihydro-3-methoxycarbonyl-6-methyl-4-m-nitrophenylpyridine-5- carboxylate (400 mg) and isopropylamine (0.5 ml) as starting materials. p-(2-Hydroxy-3-isopropylaminopropoxy)benzyl 2-cyano-1,4-dihydro-3-methoxycarbonyl-6-methyl-4-m-nitrophenylpyridine-5-carboxylate was obtained as an orange stiff foam (280 mg).
IR (KBr) 3435, 2963, 2236, 1705,1656, 1639 1612, 1586, 1529, 1514, 1438, 1350, 1248, 1213, 1176, 1093, 826, 784 cm⁻¹
¹H-NMR (CDCl₃-TMS) δ 8.02 (dd, 1H), 7.98 (d, 1H), 7.51 (2d, 1H), 7.36 (t, 1H), 7.09 (2d, 2H), 6.82 (2d, 2H), 5.146 (s, 1H), 5.07 (d, 1H), 4.91 (d, 1H), 4.09-4.03 (m, 1H), 3.98 (d, 2H), 3.747 (s, 3H), 2.93-2.85 (m, 2H), 2.79-2.73 (m, 1H), 2.415 (s, 3H), 1.12 (d, 6H);
mass spectrum, m/e (FD) 565 (M⁺+1).

### Example 17

Preparation of 2-[{p-(2-hydroxy-3-isopropylaminopropoxy)-m-methoxy}phenyl]ethyl 2-cyano-1,4-dihydro-3-methoxycarbonyl-6-methyl-4-m-nitrophenylpyridine-5-carboxylate.

The procedure described in Example 1 was followed using 2-[{p-(2,3-epoxypropoxy)-m-methoxy}phenyl]ethyl 2-cyano-1,4-dihydro-3-methoxycarbonyl-6-methyl-4-m-nitrophenylpyridine-5-carboxylate (120 mg) and isopropylamine (0.3 ml) as starting materials 2-[{p-(2-Hydroxy-3-isopropylaminopropoxy)-m-methoxy}phenyl]ethyl 2-cyano-1,4-dihydro-3-methoxycarbonyl-6-methyl-4-m-nitrophenylpyridine-5-carboxylate was obtained as an orange stiff foam (190 mg).
IR (KBr) 3306,2964, 2237, 1706, 1656, 1639, 1528, 1466, 1438, 1385, 1350, 1310, 1266, 1216, 1161, 1140, 1099, 1031, 901, 806 cm⁻¹;
¹H-NMR (CDCl₃-TMS) δ 8.05 (d, 1H), 8.036 (s, 1H), 7.50 (d, 1H), 7.39 (t, 1H), 6.82 (dd, 1H), 6.672 (s, 1H), 6.68-6.62 (m, 1H), 5.154 and 5.143 (2s, 1H), 4.32-4.26 (m, 2H), 4.07-3.95 (m, 3H), 3.81 (s, 3H), 3.78 (s, 3H), 2.90-2.74 (m, 5H), 2.323 and 2.296 (2s, 3H), 1.12-0.98 (m, 8H);
mass spectrum, m/e (FD) 609 (M⁺+1).

### Example 18

Preparation of 2-{o-(2-hydroxy-3-isopropylaminopropoxy)phenoxy}ethyl 1,4-dihydro-3-methoxycarbonyl-6-methyl-4-m-nitrophenyl-2-trifluoromethylpyridine-5-carboxylate.

The procedure described in Example 1 was followed using 2-{o-(2,3-epoxypropoxy)phenoxy}ethyl 1,4-dihydro -3-methoxycarbonyl-6-methyl-4-m-nitrophenyl-2-trifluoromethylpyridine-5-carboxylate (550 mg) and isopropylamine (1.0 ml) as starting materials. 2-{o-(2-Hydroxy-3-isopropylaminopropoxy)phenoxy}ethyl 1,4-dihydro-3-methoxycarbonyl-6-methyl-4-m- nitrophenyl-2-trifluoromethylpyridine-5-carboxylate was obtained as a yellow stiff foam (500 mg).
IR (KBr) 3345, 2964, 2873, 1708, 1628, 1530, 1505, 1457, 1350, 1282, 1256, 1224, 1180, 1128, 1086, 742 cm⁻¹;
¹H-NMR (CDCl₃-TMS) δ 8.07 (s, 1H), 7.95 (t, 1H), 7.61 (d, 1H), 7.03-7.26 (m, 1H), 6.95-6.88 (m, 3H), 6.81-6.78 (m, 1H), 6.41 (s, 1H), 5.17 (s, 1H), 4.56-4.50 (m, 1H), 4.40-4.34 (m, 1H), 4.17-3.91 (m, 7H), 3.69 (s, 3H), 2.85-2.78 (m, 2H), 2.72 (dd, 1H), 2.45 (s, 3H), 1.11-1.03 (m, 6H);
mass spectrum, m/e (FD) 638 (M⁺+1).

### Example 19

Preparation of 2-{o-(2-hydroxy-3-isopropylaminopropoxy)phenoxy}ethyl 3-ethoxycarbonyl-1,4-dihydro-6-methyl-4-m-nitrophenyl-2-trifluoromethylpyridine-5-carboxylate.

The procedure described in Example 1 was followed using 2-{o-(2,3-epoxypropoxy)phenoxy}ethyl 3-ethoxycarbonyl-1,4-dihydro-6-methyl-4-m-nitrophenyl-2- trifluoromethylpyridine-5-carboxylate (550 mg) and isopropylamine (1.0 ml) as starting materials. 2-{o-(2-Hydroxy-3-isopropylaminopropoxy)phenoxy}ethyl 3-ethoxycarbonyl-1,4-dihydro-6-methyl-4-m-nitrophenyl-2-trifluoromethylpyridine-5-carboxylate was obtained as a yellow stiff foam (500 mg).
IR (KBr) 3342, 3106, 2969, 2874, 1706, 1530, 1505, 1349, 1282, 1256, 1224, 1180, 1127, 1095, 742 cm⁻¹;
¹H-NMR (CDCl₃-TMS) δ 8.09 (dd, 1H), 7.94 (t, 1H, 7.62 (d, 1H), 7.03-7.26 (m, 1H), 6.95-6.88 (m, 3H), 6.81-6.78 (m, 1H) 6.38 (s, 1H), 5.17 (s, 1H), 4.55-4.50 (m, 1H), 4.39-4.34 (m, 1H), 4.18-3.91 (m, 7H), 2.85-2.78 (m, 2H), 2.74-2.69 (m, 1H), 2.45 (s, 3H), 1.20 (t, 3H), 1.09-1.07 (m, 6H);
mass spectrum, m/e (FD) 652 (M⁺+1).

### Example 20

Preparation of 2-{p-(2-hydroxy-3-isopropylaminopropoxy)phenoxy}ethyl 1,4-dihydro-3-methoxycarbonyl-6-methyl-4-m-nitrophenyl-2-trifluoromethylpyridine-5-carboxylate.

The procedure described in Example 1 was followed using 2-{p-(2,3-epoxypropoxy)phenoxy}ethyl 1,4-dihydro-3-methoxycarbonyl-6-methyl-4-m-nitrophenyl -2-trifluoromethylpyridine-5-carboxylate (509 mg) and isopropylamine (1.0 ml) as starting materials. 2-{p-(2-Hydroxy-3-isopropylaminopropoxy)phenoxy}ethyl 1,4-dihydro-3-methoxycarbonyl-6-methyl-4-m-nitrophenyl-2- trifluoromethylpyridine-5-carboxylate was obtained as a yellow stiff foam (450 mg).
IR (KBr) 3350, 3108, 2964, 2872, 1707, 1530, 1508, 1350, 1282, 1228, 1179, 1086, 825 cm⁻¹;
¹H-NMR (CDCl₃-TMS) δ 8.08 (t, 1H), 8.00 (dd, 1H), 7.61 (d, 1H), 7.32 (t, 1H), 6.84 (d, 2H), 6.76 (d, 2H), 6.27 (s, 1H), 5.18 (s, 1H), 4.45 (ddd, 1H), 4.33 (ddd, 1H), 4.10-4.01 (m, 3H), 3.94 (d, 2H); 3.70 (s, 3H), 2.92 (dd, 1H), 2.87 (quint., 1H), 2.75 (dd, 1H), 2.43 (s, 3H), 1.12 (d, 6H);
mass spectrum, m/e (FD) 638 (M⁺+1).

### Example 21

Preparation of 2-{p-(2-hydroxy-3-isopropylaminopropoxy)phenoxy}ethyl 3-ethoxycarbonyl-1,4-dihydro-6-methyl-4-m-nitrophenyl-2-trifluoromethylpyridine-5-carboxylate.

The procedure described in Example 1 was followed using 2-{p-(2,3-epoxypropoxy)phenoxy}ethyl 3-ethoxycarbonyl-1,4-dihydro-6-methyl-4-m-nitrophenyl-2- trifluoromethylpyridine-5-carboxylate (530 mg) and isoproylamine (1.0 ml) as starting materials. 2-{p-(2-Hydroxy-3-isopropylaminopropoxy)phenoxy}ethyl 3-ethoxycarbonyl-1,4-dihydro-6-methyl-4-m-nitrophenyl-2-trifluoromethylpyridine-5-carboxylate was obtained as a yellow stiff foam (485 mg).
IR (KBr) 3338, 3108, 2967, 1707, 1530, 1508, 1349, 1281, 1228, 1179, 1096, 824 cm⁻¹;
¹H-NMR (CDCl₃-TMS) δ 8.09 (t, 1H) 8.00 (dd, 1H), 7.62 (d, 1H), 7.31 (t, 1H), 6.84 (d, 2H), 6.75 (d, 2H), 6.24 (s, 1H), 5.17 (s, 1H), 4.44 (ddd, 1H), 4.33 (ddd, 1H), 4.18-3.39 (m, 5H), 3.94 (d, 2H) 2.92 (dd, 1H), 2.88 (quint., 1H), 2.76 (dd, 1H) 2.44 (s, 3H), 1.21 (t, 3H), 1.12 (d, 6H);
mass spectrum, m/e (FD) 652 (M⁺+1).

### Example 22

Preparation of 3-{p-(2-hydroxy-3-isopropylaminopropoxy)phenoxy}propyl 1,4-dihydro-3-methoxycarbonyl-6-methyl-4-m-nitrophenyl-2-trifluoromethylpyridine-5-carboxylate.

The procedure described in Example 1 was followed using 3-{p-(2,3-epoxypropoxy)phenoxy}propyl 1,4-dihydro-3-methoxycarbonyl-6-methyl-4-m-nitrophenyl-2-trifluoromethylpyridine-5-carboxylate (640 mg) and isopropylamine (1.0 ml) as starting materials. 3-{p-(2-Hydroxy-3-isopropylaminopropoxy)phenoxy}propyl 1,4-dihydro-3-methoxycarbonyl-6-methyl-4-m-nitrophenyl-2- trifluoromethylpyridine-5-carboxylate was obtained as a yellow stiff foam (650 mg).
IR (KBr) 3346, 2964, 1707, 1530, 1508, 1349, 1281, 1229, 1179, 1082, 825, 780, 754 cm⁻¹;
¹H-NMR (CDCl₃-TMS) δ 8.09 (t, 1H), 8.02 (d, 1H), 7.59 (d, 1H), 7.37 (t, 1H), 6.82 (d, 2H), 6.72 (d, 2H), 6.30 (s, 1H), 5.16 (s 1H), 4.32-4.15 (m, 3H), 3.99-3.92 (m, 2H), 3.80 (t, 2H), 3.71 (s, 3H), 3.03-2.98 (m, 2H), 2.84 (dd, 1H), 2.44 (s, 3H), 2.06-2.03 (m, 2H), 1.21 (d, 6H);
mass spectrum, m/e (FD) 651 (M⁺+1).

### Example 23

Preparation of 3-{p-(2-hydroxy-3-isopropylaminopropoxy)phenoxy}propyl 3-ethoxycarbonyl-1,4-dihydro-6-methyl-4-m-nitrophenyl-2-trifluoromethylpyridine-5-carboxylate.

The procedure described in Example 1 was followed using 3-{p-(2,3-epoxypropoxy)phenoxy}propyl 3-ethoxycarbonyl-1,4-dihydro-6-methyl-4-m-nitrophenyl-2- trifluoromethylpyridine-5-carboxylate (620 mg) and isopropylamine (1.0 ml) as starting materials. 3-{p-(2-Hydroxy-3-isopropylaminopropoxy)phenoxy}propyl 3-ethoxycarbonyl-1,4-dihydro-6-methyl-4-m-nitrophenyl-2- trifluoromethylpyridine-5-carboxylate was obtained as a yellow stiff foam (560 mg).
IR (KBr) 3336, 3109, 2968, 1702, 1530, 1508, 1474, 1387, 1349, 1281, 1228, 1178, 1079, 824, 779 cm⁻¹;
¹H-NMR (CDCl₃-TMS) δ 8.10 (t, 1H), 8.02 (dd, 1H), 7.60 (d, 1H), 7.37 (t, 1H), 6.82 (d, 2H), 6.72 (d, 2H), 6.25 (s, 1H), 5.16 (s, 1H), 4.31-4.12 (m, 4H), 4.07-4.02 (m, 1H), 3.94 (d, 2H), 3.81 (t, 2H), 2.93 (dd, 1H), 2.89 (quint, 1H), 2.44 (s, 3H), 2.06-2.02 (m, 2H), 1.22 (t, 3H), 1.13 (d, 6H);
mass spectrum, m/e (FD) 666 (M⁺+1).

### Example 24

Preparation of 2-{o-(2-hydroxy-3-isopropylaminopropoxy)phenoxy}ethyl 2-cyano-1,4-dihydro-3-methoxycarbonyl-6-methyl-4-m-nitrophenylpyridine-5- carboxylate.

The procedure described in Example 1 was followed using 2-{o-(2,3-epoxypropoxy)phenoxy}ethyl 2-cyano-1,4-dihydro-3-methoxycarbonyl-6-methyl-4-m-nitrophenylpyridine-5-carboxylate (400 mg) and isopropylamine (0.4 ml) as starting materials. 2-{o-(2-Hydroxy-3-isopropylaminopropoxy)phenoxy}ethyl 2-cyano-1,4-dihydro-3-methoxycarbonyl-6-methyl-4-m-nitrophenylpyridine-5-carboxylate was obtained as an orange stiff foam (400 mg).
IR (KBr) 3302, 2964, 2237, 1708, 1643, 1594, 1530, 1505, 1455, 1437, 1384, 1350, 1310, 1256, 1216, 1120, 1097, 1042, 746 cm⁻¹;
¹H-NMR (CDCl₃-TMS) δ 8.07-8.04 (m, 1H), 7.95-7.89 (m, 1H), 7.60 (t, 1H), 7.26 and 7.15 (2t, 1H), 6.87-6.77 (m, 4H), 5.191 and 5.180 (2s, 1H), 4.59-4.50 (m, 1H), 4.39-4.31 (m, 1H), 4.16-3.92 (m, 5H), 3.763 and 3.752 (2s, 3H), 2.91-2.71 (m, 3H), 2.465 and 2.442 (2s, 3H), 1.14-1.09 (m, 6H);
mass spectrum, m/e (FD) 595 (M⁺+1).

### Example 25

Preparation of 2-{p-(2-hydroxy-3-isopropylaminopropoxy)phenoxy}ethyl 2-cyano-1,4-dihydro-3-methoxycarbonyl-6-methyl-4-m-nitrophenylpyridine-5-carboxylate.

The procedure described in Example 1 was followed using 2-{p-(2,3-epoxypropoxy)phenoxy}ethyl 2-cyano-1,4-dihydro-3-methoxycarbonyl-6-methyl-4-m-nitrophenylpyridine-5-carboxylate (450 mg) and isopropylamine (0.5 ml) as starting materials. 2-{p-(2-Hydroxy-3-isopropylaminopropoxy)phenoxy}ethyl 2-cyano-1,4-dihydro-3-methoxycarbonyl-6-methyl-4-m-nitrophenylpyridine-5-carboxylate was obtained as an orange stiff foam (340 mg).
IR (KBr) 3434, 2964, 2236, 1707, 1637, 1629, 1530, 1508, 1349, 1309, 1213, 1116, 743 cm⁻¹;
⁻¹H-NMR (CDCl₃-TMS) δ 8.065 and 8.601 (2d, 1H), 8.00 (dd, 1H), 7.60 (2d, 1H), 7.32 (dt, 1H), 6.85-6.81 (m, 2H), 6.76-6.72 (m, 2H), 5.193 (s, 1H), 4.46-4.39 (m, 1H), 4.37-4.29 (m, 1H), 4.10-4.00 (m, 3H), 3.97-3.94 (m, 2H), 3.762 (s, 3H), 2.94-2.85 (m, 2H), 2.78-2.73 (m, 1H), 2.381 and 2.373 (2s, 3H), 1.13 (d, 6H);
mass spectrum, m/e (FD) 595 (M⁺+1).

### Example 26

Preparation of 3-{p-(2-hydroxy-3-isopropylaminopropoxy)phenoxy}propyl 2-cyano-1,4-dihydro-3-methoxycarbonyl-6-methyl-4-m-nitrophenylpyridine-5-carboxylate.

The procedure described in Example 1 was followed using 3-{p-(2,3-epoxypropoxy)phenoxy}propyl 2-cyano-1,4-dihydro-3-methoxycarbonyl-6-methyl-4-m-nitrophenylpyridine-5-carboxylate (860 mg) and isopropylamine (0.5 ml) as starting materials. 3-{p-(2-Hydroxy-3-isopropylaminopropoxy)phenoxy}propyl 2-cyano-1,4-dihydro-3-methoxycarbonyl-6-methyl-4-m-nitrophenylpyridine-5-carboxylate was obtained as an orange stiff foam (670 mg).
IR (KBr) 3296, 3084, 2965, 2237, 1705, 1644, 1530, 1508, 1472, 1436, 1349, 1309, 1214, 1097, 1048, 826, 752 cm⁻¹;
¹H-NMR (CDCl₃-TMS) δ 8.076 and 8.073 (2d, 1H), 8.02 (dd, 1H), 7.59 (d, 1H), 7.37 (t, 1H), 6.82-6.80 (m, 2H), 6.73-6.69 (m, 2H), 5.179 (s, 1H), 4.30-4.20 (m, 2H), 4.09-4.05 (m, 1H), 3.95 (d, 2H), 3.82-3.79 (m, 2H), 3.776 (s, 3H), 2.95-2.87 (m, 2H), 2.80-2.75 (m, 1H), 2.399 and 2.397 (2s, 3H), 2.06-2.00 (m, 2H), 1.13 (d, 6H);
mass spectrum, m/e (FD) 609 (M⁺+1).

### Example 27

Preparation of 2-{p-(2-hydroxy-3-isopropylaminopropoxy)phenylacetamido}ethyl 1,4-dihydro-3-methoxycarbonyl-6-methyl-4-m-nitrophenyl-2-trifluoromethylpyridine-5-carboxylate.

The procedure described in Example 1 was followed using 2-{p-(2,3-epoxypropoxy)phenylacetamido}ethyl 1,4-dihydro-3-methoxycarbonyl-6-methyl-4-m-nitrophenyl-2-trifluoromethylpyridine-5-carboxylate (750 mg) and isopropylamine (3.0 ml) as starting materials. 2-{p-(2-Hydroxy-3-isopropylaminopropoxy)phenylacetamido}ethyl 1,4-dihydro-3-methoxycarbonyl-6-methyl-4-m-nitrophenyl-2-trifluoromethylpyridine-5-carboxylate was obtained as a yellow stiff foam (720 mg).
IR (KBr) 3304 3092, 2964, 1708, 1654, 1526, 1516, 1437, 1386, 1350, 1224, 1176, 1082, 1037, 757 cm⁻¹;
¹H-NMR (CDCl₃-TMS) δ 8.10-8.02 (m, 2H), 7.60 (d, 1H), 7.44 (t, 1H), 7.13-7.07 (m, 2H), 6.88-6.82 (m, 2H), 5.67-5.46 (m, 1H), 5.07 (s, 1H), 4.25-3.89 (m, 5H), 3.73 (s, 3H), 3.54-3.35 (m, 4H), 2.97-2.82 (m, 2H), 2.80-2.68 (m, 1H), 2.16 (d, 3H), 1.124 (d, 6H);
mass spectrum, m/e (FD) 679 (M⁺+1)

### Example 28

Preparation of 2-{p-(2-hydroxy-3-isopropylaminopropoxy)phenylacetamido}ethyl 2-cyano-1,4-dihydro-3-methoxycarbonyl-6-methyl-4-m-nitrophenylpyridine-5-carboxylate.

The procedure described in Example 1 was followed using 2-{p-(2,3-epoxypropoxy)phenylacetamido}ethyl 2-cyano-1,4-dihydro-3-methoxycarbonyl-6-methyl-4-m- nitrophenylpyridine-5-carboxylate (770 mg) and isopropylamine (2.5 ml) as starting materials. 2-{p-(2-Hydroxy-3-isopropylaminopropoxy)phenylacetamido}ethyl 2-cyano-1,4-dihydro-3-methoxycarbonyl-6-methyl-4-m-nitrophenylpyridine-5-carboxylate was obtained as a yellow stiff foam (670 mg).
IR (KBr) 3292, 3192, 3078, 2964, 2236, 1704, 1651, 1530, 1511, 1436, 1385, 1349, 1214, 1178, 1098, 1039, 752 cm⁻¹;
¹H-NMR (CDCl₃-TMS δ 8.11-7.93 (m, 2H), 7.68-7.53 (m, 1H), 7.42 (t, 1H), 7.15-7.05 (m, 2H), 6.89-6.78 (m, 2H), 5.53-5.38 (m, 1H), 5.10 and 5.08 (2s, 1H), 4.33-3.86 (m, 5H), 3.787 and 3.782 (2s, 3H), 3.69-3.28 (m, 4H), 3.03-2.65 (m, 3H), 1.88 and 1.84 (2s, 3H), 1.34-1.08 (m, 6H);
mass spectrum, m/e (FD) 624 (M⁺+1).

### Example 29

Preparation of 2-[2-{p-(2-hydroxy-3-isopropylaminopropoxy)phenoxy}ethoxy]ethyl 2-cyano-1,4-dihydro-3-methoxycarbonyl-6-methyl-4-m-nitrophenylpyridine-5-carboxylate.

The procedure described in Example 1 was followed using 2-[2-{p-(2,3-epoxypropoxy)phenoxy}ethoxy]ethyl 2-cyano-1,4-dihydro-3-methoxycarbonyl-6-methyl-4-m-nitrophenylpyridine-5-carboxylate (460 mg) and isopropylamine (0.5 ml) as starting materials. 2-[2-{p-(2-Hydroxy-3-isopropylaminopropoxy)phenoxy}-ethoxy]ethyl 2-cyano-1,4-dihydro-3-methoxycarbonyl-6-methyl-4-m-nitrophenylpyridine-5-carboxylate was obtained as an orange stiff foam (400 mg).
IR (KBr) 3286, 3195, 3084, 1965, 2236, 1707, 1644, 1634, 1530, 1508, 1456, 1436, 1384, 1350, 1299, 1273, 1217, 1099, 1042, 826, 780, 756 cm⁻¹;
¹H-NMR (CDCl₃-TMS δ 8.08-8.03 (m, 2H), 7.62 (d, 1H), 7.40 (t, 1H), 6.82-6.79 (m, 4H), 5.142 (s, 1H), 4.30-3.97 (m, 7H), 3.82-3.68 (m, 4H), 3.754 (s, 3H), 2.99-2.78 (m, 3H), 2.288 (2s, 3H), 1.17 (d, 6H);
mass spectrum, m/e (FD) 639 (M⁺+1).

### Example 30

Preparation of 2-[2-{p-(2-hydroxy-3-isopropylaminopropoxy)phenoxy}ethoxy]ethyl 1,4-dihydro-3-methoxycarbonyl-6-methyl-4-m-nitrophenyl-2-trifluoromethylpyridine-5-carboxylate monohydrochloride.

The procedure described in Example 5 was followed using 2-[2-{p-(2,3-epoxypropoxy)phenoxy}ethoxy)ethyl 1,4-dihydro-3-methoxycarbonyl-6-methyl-4-m-nitrophenyl-2-trifluoromethylpyridine-5-carboxylate (680 mg) and isopropylamine (1.0 ml) as starting materials. 2-[2-{p-(2-Hydroxy-3-isopropylaminopropoxy)phenoxy}ethoxy]ethyl 1,4-dihydro-3-methoxycarbonyl-6-methyl-4-m-nitrophenyl-2-trifluoromethylpyridine-5-carboxylate monohydrochloride was obtained as a yellow stiff foam (680 mg).
IR (KBr) 3344, 2952, 1707, 1651, 1626, 1530, 1508, 1350, 1229, 1178, 1084, 826 cm⁻¹;
¹H-NMR (CDCl₃-TMS) δ 961 (Br s, 1H), 8.47 (br s, 1H), 8.10 (s, 1H), 8.03 (d, 1H), 7.65 (d, 1H), 7.40 (t, 1H), 6.79 (br s, 4H), 6.53 (br s, 1H), 5.17 (s, 1H), 4.61, (br s, 1H), 4.27-4.19 (m, 2H), 4.03-3.95 (m, 4H), 3.78-3.70 (m, 2H), 3.69 (s, 3H), 3.45 (br s, 1H), 3.31 (br s, 1H), 3.17 (br s, 1H), 2.41 (s, 3H), 1.50 (d, 3H), 1.49 (d, 3H);
mass spectrum m/e (FD) 682 (M⁺+1).

### Example 31

Preparation of 2-[N-[2-{p-(2-hydroxy-3-isopropylaminopropoxy)}phenyl]ethyl-N-methyl]aminoethyl 1,4-dihydro-3-methoxycarbonyl-6-methyl-4-m-nitrophenyl-2-trifluoromethylpyridine-5-carboxylate dihydrochloride.

The procedure described in Example 5 was followed using 2-[N-[2-{p-(2,3-epoxypropoxy)}phenyl]ethyl-N-methyl]aminoethyl 1,4-dihydro-3-methoxycarbonyl-6-methyl-4-m-nitrophenyl-2-trifluoromethylpyridine-5-carboxylate (610 mg) and isopropylamine (0.6 ml) as starting materials. 2-[N-[2-{p-(2-Hydroxy-3-isopropylaminopropoxy)}phenyl]ethyl-N-methyl]aminoethyl 1,4-dihydro-3-methoxycarbonyl-6-methyl-4-m-nitrophenyl-2-trifluoromethylpyridine-5-carboxylate dihydrochloride was obtained as a yellow stiff foam (600 mg).
IR (KBr) 3462, 2954, 1708, 1652, 1616, 1530, 1514, 1350, 1281, 1226, 1180, 1085, 825 cm⁻¹;
¹H-NMR (CD₃OD-TMS) δ 8.09 (br t, 1H), 8.06 (br d, 1H), 7.67 (br d, 1H), 7.53 (t, 1H), 7.23-7.15 (m, 2H), 6.94 (d, 2H), 5.12 and 5.11 (2s, 1H), 4.53-4.39 (m, 2H), 4.27-4.21 (m, 1H), 4.07-3.98 (m, 2H), 3.69 (s, 3H), 3.68-3.25 (m, 5H), 3.14 (t, 2H), 2.96 (br s, 2H), 2.91 (s, 3H), 2.47 (s, 3H), 1.38 and 1.37 (2d, 6H);
mass spectrum, m/e (FD) 679 (M⁺+1).

### Example 32

Preparation of 3-{p (2-hydroxy-3-isopropylaminopropoxy)phenyl}propyl 4-o-chlorophenyl-1,4-dihydro-3-methoxycarbonyl-6-methyl-2-trifluoromethylpyridine-5-carboxylate.

The procedure described in Example 1 was followed using 3-{p-(2,3-epoxypropoxy)phenyl}propyl 4-o-chlorophenyl-1,4-dihydro-3-methoxycarbonyl-6-methyl-2-trifluoromethylpyridine-5-carboxylate (720 mg) and isopropylamine (1.0 ml) as starting materials. 3-{p-(2-Hydroxy-3-isopropylaminopropoxy)phenyl}propyl 4-o-chlorophenyl-1,4-dihydro-3-methoxycarbonyl-6-methyl-2-trifluoromethylpyridine-5-carboxylate was obtained as a yellow stiff foam (560 mg).
IR (KBr) 3342, 2960, 1732, 1702, 1626, 1513, 1355, 1283, 1223, 1178, 1140, 1098, 1075, 1036, 830, 753 cm⁻¹;
¹H-tMR (CDCl_{S}-TMS) δ 7.34 (dd, 1H), 7.28 (dd, 1H), 7.21 (t, 1H), 7.12 (t, 1H), 6.97 (d, 2H), 6.81 (d, 2H), 5.91 (s, 1H), 5.50 (s, 1H), 4.05-3.90 (m, 5H), 3.69 (s, 3H), 2.93 (dd, 1H), 2.89 (quint. 1H), 2.76 (dd, 1H), 2.44-2.40 (m, 2H), 2.42 (s, 3H), 1.86-1.81 (m, 2H), 1.13 (d, 6H);
mass spectrum, m/e (FD) 625 (M⁺+1)

### Example 33

Preparation of 3-{p-(2-hydroxy-3-isopropylaminopropoxy)phenyl}propyl 1,4-dihydro-3-methoxycarbonyl-6-methyl-4-o-nitrophenyl-2-trifluoromethylpyridine-5-carboxylate.

The procedure described in Example 1 was followed using 3-{p-(2,3-epoxypropoxy)phenyl}propyl 1,4-dihydro-3-methoxycarbonyl-6-methyl-4-o-nitrophenyl-2-trifluoromethylpyridine-5-carboxylate (660 mg) and isopropylamine (1.0 ml) as starting materials. 3-{p-(2-hydroxy-3-isopropylaminopropoxy)phenyl}propyl 1,4-dihydro-3-methoxycarbonyl-6-methyl-4-o-nitropnenyl-2-trifluoromethylpyridine-5-carboxylate was obtained as a yellow stiff foam (540 mg).
IR (KBr) 3342, 2959, 1735, 1705, 1627, 1530, 1512, 1356, 1281, 1243, 1181, 1143;1097, 858, 829, 746, 712 cm⁻¹;
¹H-NMR (CDCl₃-TMS) δ 7.76 (d, 1H), 7.57-7.52 (m, 2H), 7.32 (t, 1H), 6.99 (d, 2H), 6.80 (d, 2H), 5.95 (s, 1H), 5.73 (s, 1H), 4.11-3.92 (m, 5H), 3.70 (s, 3H) 2.99-2.91(m, 2H), 2.82-2.78 (m, 1H), 2.42 (s, 3H), 2.43-2.38 (m, 2H), 1.79 (quint, 2H), 1.17 (d, 6H);
mass spectrum, m/e (FD) 636 (M⁺+1).

### Example 34

Preparation of 2-{p-(2-hydroxy-3-isopropylaminopropoxy)phenoxy}ethyl 4-o-chlorophenyl-1,4-dihydro-3-methoxycarbonyl-6-methyl-2-trifluoromethylpyridine-5-carboxylate.

The procedure described in Example 1 was followed using 2-{p-(2,3-epoxypropoxy)phenoxy}ethyl 4-o-chlorophenyl-1,4-dihydro-3-methoxycarbonyl-6-methyl-2-trifluoromethylpyridine-5-carboxylate (607 mg) and isopropylamine (1.0 ml) as starting materials. 2-{p-(2-Hydroxy-3-isopropylaminopropoxy)phenoxy}ethyl 4-o-chlorophenyl-1,4-dihydro-3-methoxycarbonyl-6-methyl-2-trifluoromethylpyridine-5-carboxylate was obtained as a yellow stiff foam (460 mg).
IR (KBr) 3342, 2964, 1706, 1509, 1283, 1231, 1180, 1098, 1078, 1037, 824, 754 cm⁻¹;
¹H-NMR (CDCl₃-TMS) δ 7.33 (dd, 1H), 720 (dd, 1H), 7.15 (t, 1H), 7.0 (t, 1H), 6.84 (d, 2H), 6.74 (d, 28), 5.95 (s, 1H), 5.49 (s, 1H), 4.31 (t, 2H), 4.06-3.98 (m, 3H), 3.94 (dd, 2H), 3.65 (s, 3H), 2.92 (dd, 1H), 2.88 (quint. 1H), 2.75 (dd, 1H), 2.40 (s, 3H), 1.12 (d, 6H);
mass spectrum, m/e (FD) 627 (M⁺+1).

### Example 35

Preparation of 2-{p-(2-hydroxy-3-isopropylaminopropoxy)phenyl}ethyl 4-o-chlorophenyl-1,4-dihydro-3-methoxycarbonyl-6-methyl-2-trifluoromethylpyridine-5-carboxylate.

The procedure described in Example 1 was followed using 2-{p-(2,3-epoxypropoxy)phenyl}ethyl 4-o-chlorophenyl-1,4-dihydro-3-methoxycarbonyl-6-methyl-2-trifluoromethylpyridine-5-carboxylate (800 mg) and isopropylamine (1.0 ml) as starting materials. 2-{p-(2-Hydroxy-3-isopropylaminopropoxy)phenyl}ethyl 4-o-chlorophenyl-1,4-dihydro-3-methoxycarbonyl-6-methyl-2-trifluoromethylpyridine-5-carboxylate was obtained as a yellow stiff foam (490 mg).
IR (Mr) 3342, 2963, 1702, 1628, 1513, 1353, 1282, 1224, 1179, 1140, 1097, 1078, 1036, 753 cm⁻¹;
¹H-NMR (CDCl₃-TMS) δ 7.30 (dd, 1H), 7.29 (dd, 1H), 7.20 (t, 1H), 7.13 (t, 1H), 7.00 (d, 2H), 6.79 (d, 2H), 5.92 (s, 1H), 5.48 (s, 1H) 4.18 (t, 2H), 4.07-4.06 (m, 1H), 3.98-3.93 (m 2H), 3.67 (s, 3H), 2.92 (dd, 1H), 2.89 (quint, 1H), 2.84-2.37 (m, 3H), 2.34 (s, 3H), 1.13 (d, 6H);
mass spectrum, m/e (FD) 610 (M⁺+1).

### Example 36

Preparation of 2-{p-(2-hydroxy-3-isopropylaminopropoxy)phenoxy}ethyl 1,4-dihydro-3-methoxycarbonyl-6-methyl-4-o-nitrophenyl-2-trifluoromethylpyridine-5-carboxylate.

The procedure described in Example 1 was followed using 2-{p-(2,3-epoxypropoxy)phenoxy}ethyl 1,4-dihydro-3-methoxycarbonyl-6-methyl-4-o-nitrophenyl-2-trifluoromethylpyridine-5-carboxylate (790 mg) and isopropylamine (1.0 ml) as starting materials. 2-{p-(2-hydroxy-3-isopropylaminopropoxy)phenoxy}ethyl 1,4-dihydro-3-methoxycarbonyl-6-methyl-4-o-nitrophenyl-2-trifluoromethylpyridine-5-carboxylate was obtained as a yellow stiff foam (550 mg).
IR (KBr) 3341, 2963, 1732, 1707, 1530, 1508, 1355, 1231, 1181, 1143, 1082, 826 cm⁻¹;
¹H-NMR 9CDCl₃-TMS) δ 7.66 (d, 1H), 7.52 (d, 1H), 7.30-7.26 (m, 1H), 6.82 (d, 2H), 6.71 (d, 2H), 5.96 (s, 1H), 5.75 (s, 1H), 4.34-3.30 (m, 1H), 4.28-4.24 (m, 1H), 4.12-4.06 (m, 1H), 3.97 (t, 2H), 3.94 (t, 2H), 3.67 (s, 3H), 2.96 (dd, 1H), 2.93 (quint, 1H), 2.79 (dd, 1H), 2.34 (s, 3H), 1.16 (d, 6H);
mass spectrum, m/e FD) 638 (M⁺+1).

### Example 37

Preparation of 2-{p-(2-hydroxy-3-isopropylaminopropoxy)phenyl}ethyl 1,4-dihydro-3-methoxycarbonyl-6-methyl-4-o-nitrophenyl-2-trifluoromethylpyridine-5-carboxylate.

The procedure described in Example 1 was followed using 2-{p-(2,3-epoxypropoxy)phenyl}ethyl 1,4-dihydro-3-methoxycarbonyl-6-methyl-4-o-nitrophenyl-2-trifluoromethylpyridine-5-carboxylate (808 mg) and isopropylamine (1.0 ml) as starting materials. 2-{p-(2-Hydroxy-3-isopropylaminopropoxy)phenyl}ethyl 1,4-dihydro-3-methoxycarbonyl-6-methyl-4-o-nitrophenyl-2-trifluoromethylpyridine-5-carboxylate was obtained as a yellow stiff foam (494 mg).
IR (KBr) 3341, 2962, 1732, 1705, 1530, 1513, 1356, 1281, 1243, 1181, 1144, 1094, 782 cm⁻¹;
¹H-NMR (CDCl₃-TMS) δ 7.74 (d, 1H), 7.57-7.50 (m, 2H), 7.34 (t, 1H), 6.99 (d, 2H), 6.78 (d, 2H), 5.93 (s, 1H), 5.71 (s, 1H), 4.19-4.05 (m, 3H), 3.98-3.93 (m, 2H), 3.70 (s, 3H), 2.95 (dd, 1H), 2.92 (quint. 1H), 2.78 (dd, 1H), 2.73 (t, 2H), 2.35 (s, 3H), 1.15 (d, 6H);
mass spectrum, m/e (FD) 622 (M⁺+1).

### Example 38

Preparation of 4-[2-{p-(2-hydroxy-3-isopropylaminopropoxy)}phenylethyl]piperidino 1,4-dihydro-3-methoxycarbonyl-6-methyl-4-m-nitrophenyl-2-trifluoromethylpyridine-5-carboxylate dihydrochloride.

A mixture of 1,4-dihydro-3-methoxycarbonyl-6-methyl-4-m-nitrophenyl-2-trifluoromethylpyridine-5-carboxylic acid (950 mg), 1-[2-[p-{5-(3-isopropyl-2-phenyl)oxazolinyl}methoxy]phenylethyl]piperidin-4-ol (860 mg), N,N'-dicyclohexylcarbodiimide (690 mg) and 4-dimethylaminopyridine (310 mg) in dichloromethane (25 ml) was stirred at room temperature for 2h. The reaction mixture was washed with 10% NH₄Cl aq. and brine, dried over MgSO₄, and concentrated under reduced pressure. The residue was chromatographed using a silica gel column (chloroform/methanol=9/1 v/v). 4-[2-{p-(2-Hydroxy-3-isopropylaminopropoxy)}phenylethyl]piperidino 1,4-dihydro-3-methoxycarbonyl-6-methyl-4-m-nitrophenyl-2-trifluoromethylpyridine-5-carboxylate was obtained as a yellow foam (690 mg).

To a solution of 4-[2-{p-(2-hydroxy-3-isopropylaminopropoxy)}phenylethyl]piperidino 1,4-dihydro-3-methoxycarbonyl-6-methyl-4-m-nitrophenyl-2-trifluoromethylpyridine-5-carboxylate in methanol (3.0 ml) was added 2N methanol solution of hydrogen chloride (2.0 ml). The reaction mixture was stirred for 1 h at room temperature. Methanol was evaporated under reduced pressure. 4-[2-{p-(2-Hydroxy-3-isopropylaminopropoxy)}phenylethyl]piperidino 1,4-dihydro-3-methoxycarbonyl-6-methyl-4-m-nitrophenyl-2-trifluoromethylpyridine-5-carboxylate dihydrochloride was obtained as a yellow stiff foam (720 mg).
IR (KBr) 3350, 2954, 2703, 1705, 1616, 1532, 1526, 1522, 1514, 1510, 1352, 1341, 1280, 1244, 1222, 1216, 1176, 1160, 1086 cm⁻¹;
¹H-NMR (CD₃COCD₃-TMS) δ 8.66 (br s, 1H), 8.17 (s, 1H), 8.10 (br s, 1H), 8.02 (s, 1H), 7.82 (br d, 1H), 7.66 (t, 1H), 7.21 (d, 2H), 6.85 (br d, 2H), 5.79 (br s, 1H), 5.19 (s, 1H), 5.03 (br s, 1H), 4.51 (br d, 2H), 4.00 (ddd, 2H), 3.69 (s, 3H), 3.65-1.95 (m, 15H), 2.53 (s, 3H), 1.49 and 1.48 (2d, 6H);
mass spectrum m/e (FD) 705 (M⁺+1).

### Example 39

Preparation of 2-{p-(2-hydroxy-3-t-butylaminopropoxy)phenoxy}ethyl 2-cyano-1,4-dihydro-3-methoxycarbonyl-6-methyl-4-m-nitrophenylpyridine-5-carboxylate.

The procedure described in Example 1 was followed using 2-{p-(2,3-epoxypropoxy)phenoxy}ethyl 2-cyano-1,4-dihydro-3-methoxycarbonyl-6-methyl-4-m-nitrophenylpyridine-5-carboxylate (600 mg) and t-butylamine (0.8 ml) as starting materials. 2-{p-(2-Hydroxy-3-t-butylaminopropoxy)phenoxy}ethyl 2-cyano-1,4-dihydro-3-methoxycarbonyl-6-methyl-4-m-nitro-phenylpyridine-5-carboxylate was obtained as an orange stiff foam (380 mg).
IR (KBr) 3300, 3084, 2964, 2237, 1708, 1644, 1530, 1508, 1456, 1350, 1310, 1274, 1212, 1118, 1074, 1041, 826, 754 cm⁻¹;
¹H-NMR (CDCl₃-TMS) δ 8.065 and 8.602 (2d, 1H), 7.99 (2dd, 1H), 7.60 (d, 1H), 7.31 (t, 1H), 6.82 (d, 2H), 6.73 (2d, 2H), 5.184 (s, 1H), 4.45-4.30 (m, 2H), 4.10-3.94 (m, 5H), 3.753 (s, 3H), 2.94 (dd, 1H), 2.80-2.75 (m, 1H), 1.194 (s, 9H);
mass spectrum, m/e (FD) 609 (M⁺+1).

### Example 40

Preparation of 4-[2-{p-(2-hydroxy-3-isopropylaminopropoxy)phenyl}ethyl]piperidino 2-cyano-1,4-dihydro-3-methoxycarbonyl-6-methyl-4-m-nitrophenylpyridine-5-carboxylate.

The procedure described in Example 38 was followed using 2-cyano-1,4-dihydro-3-methoxycarbonyl-6-methyl-4-m-nitrophenylpyridine-5-carboxylic acid (480 mg), 1-[2-[p-{5-(3-isopropyl-2-phenyl)oxyazolinyl}methoxy]phenylethyl]piperidin-4-ol (580 mg), N,N'-dicyclohexylcarbodiimide (430 mg) and 4-dimethylaminopyridine (190 mg) as starting materials. 4-[2-{p-(2-Hydroxy-3-isopropylaminopropoxy)phenyl}ethyl]piperidino 2-cyano-1,4-dihydro-3-methoxycarbonyl-6-methyl-4-m-nitro-phenylpyridine-5-carboxylate was obtained as an orange stiff foam (420 mg).
IR (KBr) 2954, 2236, 1705, 1530, 1512, 1349, 1215, 1096, 826, 783, 734 cm⁻¹;
¹H-NMR (CDCl₃-TMS) δ 8.11 (t, 1H), 8.09-8.06 (m, 1H), 7.63 (d, 1H), 7.44 (t, 1H), 7.10 (d, 2H), 6.84 (d, 2H), 5.19 (s, 1H), 4.80-4.74 (m, 1H), 4.09-4.04 (m, 1H), 3.96 (d, 2H), 3.78 (s, 3H), 2.94-2.84 (m, 2H), 2.78-2.49 (m, 7H), 2.43 (s, 3H), 2.33-2.20 (m, 2H), 1.96-1.85 (m, 1H), 1.80-1.65 (m, 2H), 1.63-1.55 (m, 1H), 1.12 (d, 6H);
mass spectrum, m/e (FD) 662 (M⁺+1).

### Example 41

Preparation of p-(2-hydroxy-3-isopropylaminopropoxy)benzyl 1,4-dihydro-3-methoxycarbonyl-6-methyl-4-o-nitrophenyl-2-trifluoromethylpyridine-5-carboxylate.

The procedure described in Example 1 was followed using p-(2,3-epoxypropoxy)benzyl 1,4-dihydro-3-methoxycarbonyl-6-methyl-4-o-nitrophenyl-2-trifluoromethylpyridine-5-carboxylate (550 mg) and isopropylamine (1.0 ml) as starting materials. p-(2-Hydroxy-3-isopropylaminopropoxy)benzyl 1,4-dihydro-3-methoxycarbonyl-6-methyl-4-o-nitrophenyl-2-trifluoromethylpyridine-5-carboxylate was obtained as a yellow stiff foam (510 mg).
IR (KBr) 3338, 2963, 1733, 1705, 1530, 1514, 1356, 1280, 1243, 1177, 1049, 829, 782 cm⁻¹;
¹H-NMR (CDCl₃-TMS) δ 7.73 (d, 1H), 7.56-7.49 (m, 2H), 7.33 (t, 1H), 7.09 (d, 1H), 6.79 (d, 2H), 5.90 (s, 1H), 5.71 (s, 1H), 4.98 (d, 1H), 4.97 (d, 1H), 4.07-4.03 (m, 1H), 3.98-3.92 (m, 2H), 3.68 (s, 3H), 2.93 (dd, 1H), 2.89 (quint. 1H), 2.75 (dd, 1H), 2.38 (s, 3H), 1.13 (d, 6H);
mass spectrum, m/e (FD) 608 (M⁺+1).

### Example 42

Preparation of 3-[2-{p-(2-hydroxy-3-isopropylaminopropoxy)phenyl}ethyl]piperidino 1,4-dihydro-3-methoxycarbonyl-6-methyl-4-m-nitrophenyl-2-trifluoromethylpyridine-5-carboxylate dihydrochloride.

The procedure described in Example 38 was followed using 1,4-dihydro-3-methoxycarbonyl-6-methyl-4-m-nitrophenyl-2-trifluoromethylpyridine-5-carboxylic acid (620 mg), 1-[2-[p-{5-(3-isopropyl-2-phenyl)-oxazolinyl}methoxy]phenylethyl]piperidin-3-ol (680 mg), N,N'-dicyclohexylcarbodiimide (500 mg) and 4-dimethylaminopyridine (220 mg) as starting materials. 3-[2-{p-(2-Hydroxy-3-isopropylaminopropoxy)phenyl}ethyl]piperidino 1,4-dihydro-3-methoxycarbonyl6-methyl-4-m-nitrophenyl-2-trifluoromethylpyridine-5-carboxylate dihydrochloride was obtained as a pale yellow stiff foam (450 mg).
IR (KBr) 3348, 2951, 1704, 1530, 1512, 1349, 1279, 1229, 1179, 1096, 827, 756 cm⁻¹;
¹H-NMR (CDCl₃-TMS ) δ 8.16-8.05 (m, 2H), 7.68-7.63 (m, 1H), 7.46-7.41 (m, 1H), 7.09-7.04 (m, 2H), 6.82-6.74 (m, 2H), 6.53, 6.49 and 6.38 (3br s, 1H), 5.16 (s, 1H), 4.89-4.81 (m, 1H), 4.45 (br s, 1H) 4.06-3.93 (m, 2H), 3.71 and 3.70 (2s, 3H), 3.70-3.63 (m, 1H), 3.29 (br s, 1H), 3.21 (br d, 1H), 3.08-3.00 (m, 1H), 2.70-2.45 (m, 5H), 2.40, 2.394, 2.391 and 2.38 (4s 3H), 2.36-1.50 (m, 3H), 1.40 and 1.39 (2d, 6H);
mass spectrum, m/e (FD) 705 (M⁺+1).

### Example 43

Preparation of 3-[2-{p-(2-hydroxy-3-isopropylaminopropoxy)phenyl}ethyl]piperidino 2-cyano-1,4-dihydro-3-methoxycarbonyl-6-methyl-4-m-nitrophenylpyridine-5-carboxylate.

The procedure described in Example 38 was followed using 2-cyano-1,4-dihydro-3-methoxycarbonyl-6-methyl-4-m-nitrophenylpyridine-5-carboxylic acid (580 mg), 1-[2-[p-{5-(3-isopropyl-2-phenyl)oxazolinyl}methoxy]phenylethyl]piperidin-3-ol (690 mg) N,N'-dicyclohexylcarbodiimide (510 mg) and 4-dimethylaminopyridine (220 mg) as starting materials. 3-[2-{p-(2-Hydroxy-3-isopropylaminopropoxy)phenyl}ethyl]piperidino 2-cyano-1,4-dihydro-3-methoxycarbonyl-6-methyl-4-m-nitrophenylpyridine-5-carboxylate was obtained as an orange stiff foam (450 mg).
IR (KBr) 2951, 2236, 1706, 1530, 1512, 1349, 1217, 1116, 1097, 754 cm⁻¹;
¹H-NMR (CDCl₃-TMS) δ 8.12-8.03 (m, 2H), 7.69-7.60 (m, 1H), 7.47-7.39 (m, 1H), 7.07-6.98 (m, 2H), 6.75 (t, 2H), 5.26, 5.25, 5.17 and 5.16 (4s, 1H), 4.93-4.78 (m, 1H), 4.20-3.95 (m, 3H), 3.78, 3.766 and 3.764 (3s, 3H), 3.10-2.10 (m, 11H), 2.10, 2.05, 1.95 and 1.92 (4s, 3H), 1.75-1.45 (m, 4H), 1.22-1.12 (m, 6H);
mass spectrum, m/e (Fd) 662 (M⁺+1).

### Example 44

Preparation of 4-{p-(2-hydroxy-3-isopropylaminopropoxy)phenoxy}butyl 2-cyano-1,4-dihydro-3-methoxycarbonyl-6-methyl-4-m-nitrophenylpyridine-5-carboxylate.

The procedure described in Example 1 was followed using 4-{p-(2,3-epoxypropoxy)phenoxy}butyl 2-cyano-1,4-dihydro-3-methoxycarbonyl-6-methyl-4-m-nitrophenylpyridine-5-carboxylate (1110 mg) and isopropylamine (0.5 ml) as starting materials. 4-{p-(2-Bydroxy-3-isopropylaminopropoxy)phenoxy}butyl 2-cyano-1,4-dihydro-3-methoxycarbonyl-6-methyl-4-m-nitrophenylpyridine-5-carboxylate was obtained as an orange stiff foam (1100 mg).
IR (KBr) 3296, 3084, 2962, 2236, 1707, 1642, 1530, 1508, 1349, 1309, 1216, 1097, 1040, 825, 751 cm⁻¹;
¹H-NMR (CDCl₃-TMS) δ 8.078 (s, 1H), 8.06 (d, 1H), 7.61 (d, 1H), 7.42 (t, 1H), 6.84-6.75 (m, 4H), 5.164 and 5.156 (2s, 1H), 4.15-3.89 (m, 7H), 3.780 (s, 3H), 2.95-2.73 (m, 3H), 2.323 and 2.319 (2s, 3H), 1.81-1.67 (m, 4H), 1.14 (d, 6H);
mass spectrum, m/e (Fd) 623 (M⁺+1).

### Example 45

Preparation of p-(2-hydroxy-3-isopropylaminopropoxy)phenylmethyl 4-m-fluorophenyl-1,4-dihydro-3-methoxycarbonyl-6-methyl-2-trifluoromethylpyridine-5-carboxylate monohydrochloride.

The procedure described in Example 5 was followed using p-(2,3-epoxypropoxy)phenylmethyl 4-m-fluorophenyl-1,4-dihydro-3-methoxycarbonyl-6-methyl-2-trifluoromethylpyridine-5-carboxylate (320 mg) and isopropylamine (0.6 ml) as starting materials. p-(2-Hydroxy-3-isopropylaminopropoxy)phenylmethyl 4-m-fluorophenyl-1,4-dihydro-3-methoxycarbonyl-6-methyl-2-trifluoromethylpyridine-5-carboxylate monohydrochloride was obtained as a pale yellow stiff foam (250 mg).
IR (KBr) 3350, 2984, 1704, 1613, 1588, 1515, 1487, 1347, 1280, 1226, 1177, 1078 cm⁻¹;
¹H-NMR (CDCl₃-TMS) δ 9.75 (br s, 1H), 8.52 (br s, 1H), 7.22-7.15 (m, 1H), 7.12 (d, 1H), 6.92-6.82 (m, 5H), 6.10 (br s, 1H), 5.03 (s, 1H), 5.01 (dd, 2H), 4.64 (br s, 1H), 4.13-3.97 (m, 2H), 3.68 (s, 3H), 3.50-3.30 (m, 2H), 3.20-3.11 (m, 1H), 2.39 (s, 3H), 1.52 and 1.51 (2d, 6H);
mass spectrum, m/e (FD) 581 (M⁺+1).

### Example 46

Preparation of p-(2-hydroxy-3-isopropylaminopropoxy)phenylmethyl 4-o-fluorophenyl-1,4-dihydro-3-methoxycarbonyl-6-methyl-2-trifluoromethylpyridine-5-carboxylate monohydrochloride.

The procedure described in Example 5 was followed using p-(2,3-epoxypropoxy)phenylmethyl 4-o-fluorophenyl-1,4-dihydro-3-methoxycarbonyl-6-methyl-2-trifluoromethylpyridine-5-carboxylate (600 mg) and isopropylamine (1.2 ml) as starting materials. p-(2-Hydroxy-3-isopropylaminopropoxy)phenylmethyl 4-o-fluorophenyl-1,4-dihydro-3-methoxycarbonyl-6-methyl-2-trifluoromethylpyridine-5-carboxylate monohydrochloride was obtained as a pale yellow stiff foam (320 mg).
IR (KBr) 3366, 2983, 2954, 1734, 1700, 1615, 1515, 1488, 1279, 1255, 1177, 1093, 1074, 759 cm⁻¹;
¹H-NMR (CDCl₃-TMS) δ 9.60 (br s, 1H), 8.57 (br s, 1H), 7.26-6.78 (m, 8H), 6.18 (br s, 1H), 5.26 (s, 1H), 4.96 (dd, 2H), 4.66 (br s, 1H), 4.13-3.97 (m, 2H), 3.64 (s, 3H), 3.51-3.13 (m, 3H), 2.30 (s, 3H), 1.50 and 1.49 (2d, 6H);
mass spectrum, m/e (FD) 581 (M⁺+1).

### Example 47

Preparation of 2-[4-[2-{p-(2-hydroxy-3-isopropylaminopropoxy)phenyl}ethyl]piperazin-1-yl]ethyl 1,4-dihydro-3-methoxycarbonyl-6-methyl-4-m-nitrophenyl-2-trifluoromethylpyridine-5-carboxylate trihydrochloride.

The procedure described in Example 38 was followed using 1,4-dihydro-3-methoxycarbonyl-6-methyl-4-m-nitrophenyl-2-trifluoromethylpyridine-5-carboxylic acid (770 mg), 2-[4-[2-[p-{5-(3-isopropyl-2-phenyl)oxazolinyl)methoxy]phenylethyl]piperazine-1-yl]ethanol (920 mg), N,N'-dicyclohexylcarbodiimide (450 mg) and 4-dimethylaminopyridine (290 mg) as starting materials. 2-[4-[2-{p-(2-Hydroxy-3-isopropylaminopropoxy)phenyl}ethyl]piperazin-1-yl]ethyl 1,4-dihydro-3-methoxycarbonyl-6-methyl-4-m-nitrophenyl-2-trifluoromethylpyridine-5-carboxylate trihydrochloride was obtained as a pale yellow stiff foam (330 mg).
IR (KBr) 3400, 2954, 1708, 1529, 1514, 1438, 1351, 1281, 1211, 1181, 1152, 1086, 756 cm⁻¹;
¹H-NMR (D₂O-TMS) δ 8.14 (t, 2H), 7.78 (d, 1H), 7.59 (t, 1H), 7.31 (d, 2H), 7.04 (d, 2H), 5.11 (s, 1H), 4.38-4.30 (m, 3H), 4.19-4.09 (m, 2H), 3.76 (s, 3H), 3.55-3.01 (m, 17H), 2.43 (s, 3H), 1.37 and 1.36 (2d, 6H);
mass spectrum, m/e (Fd) 734 (M⁺+1).

### Example 48

Preparation of 2-[4-[2-{p-(2-hydroxy-3-isopropylaminopropoxy)phenyl}ethyl]piperazin-1-ethyl 2-cyano-1,4-dihydro-3-methoxycarbonyl-6-methyl-4-m-nitrophenylpyridine-5-carboxylate.

The procedure described in Example 38 was followed using 2-cyano-1,4-dihydro-3-methoxycarbonyl-6-methyl-4-m-nitrophenylpyridine-5-carboxylic acid (820 mg), 2-[4-[2-[p-{5-(3-isopropyl-2-phenyl)-oxazolinyl}methoxy]phenylethyl]piperazin-1-yl]ethanol (1080 mg), N,N'-dicyclohexylcarbodiimide (540 mg) and 4-dimethylaminopyridine (370 mg) as starting materials. 2-[4-[2-{p-(2-Hydroxy-3-isopropylaminopropoxy)phenyl}ethyl]piperazin-1-yl] ethyl 2-cyano-1,4-dihydro-3-methoxycarbonyl-6-methyl-4-m-nitrophenylpyridine-5-carboxylate was obtained as an orange stiff foam (700 mg).
IR (KBr) 2960, 2820, 2236, 1707, 1530, 1512, 1349, 1298, 1272, 1246, 1216, 1101, 826 cm⁻¹;
¹H-NMR (CDCl₃-TMS) δ 8.11-8.07 (m, 2H), 7.64 (d, 1H), 7.44 (t, 1H), 7.11 (d, 2H), 6.83 (d, 2H), 5.21 (s, 1H), 4.22-4.12 (m, 3H), 3.98 (t, 2H), 3.78 (s, 3H), 3.02-2.40 (m, 17H), 2.43 (s, 3H), 1.18 (d, 6H);
mass spectrum, m/e (FD) 691 (M⁺+1).

### Example 49

Preparation of 5-{p-(2-hydroxy-3-isopropylaminopropoxy)phenoxy}pentyl 2-cyano-1,4-dihydro-3-methoxycarbonyl-6-methyl-4-m-nitrophenylpyridine-5-carboxylate.

The procedure described in Example 1 was followed using 5-{p-(2,3-epoxypropoxy)phenoxy}pentyl 2-cyano-1,4-dihydro-3-methoxycarbonyl-6-methyl-4-m-nitrophenylpyridine-5-carboxylate (1180 mg) and isopropylamine (1.0 ml) as starting materials. 5-{p-(2-Hydroxy-3-isopropylaminopropoxy)phenoxy}pentyl 2-cyano-1,4-dihydro-3-methoxycarbonyl-6-methyl-4-m-nitrophenylpyridine-5-carboxylate was obtained as an orange stiff foam (1020 mg).
IR (KBr) 3626, 3300, 3088,. 2953, 2868, 2236, 1706, 1644, 1530, 1507, 1472, 1436, 1386, 1349, 1309, 1272, 1216, 1097, 1031, 826, 783, 745 cm⁻¹;
¹H-NMR (CDCl₃-TMS) δ 8.08 (dd, 1H), 8.05 (dd, 1H), 7.61 (dd, 1H), 7.41 (t, 1H), 6.85-6.76 (m, 4H), 5.169 (s, 1H), 4.13-4.06 (m, 3H), 3.97-3.95 (m, 2H), 3.90-3.85 (m, 2H), 3.775 (s, 3H), 2.99-2.75 (m, 3H), 2.370 (s, 3H), 1.75-1.60 (m, 4H), 1.46-1.38 (m, 2H), 1.19-1.13 (m, 6H);
mass spectrum, m/e (Fd) 637 (M⁺+1).

### Example 50

Preparation of 6-[N-{p-(2-hydroxy-3-isopropylaminopropoxy)phenyl}amino]hexyl 1,4-dihydro-3-methoxycarbonyl-6-methyl-4-m-nitrophenyl-2-trifluoromethylpyridine-5-carboxylate dihydrochloride.

A mixture of 6-bromohexyl 1,4-dihydro-3-methoxycarbonyl-6-methyl-4-m-nitrophenyl-2-trifluoromethylpyridine-5-carboxylate (700 mg) and p-(2-hydroxy-3-isopropylaminopropoxy)aniline (2750 mg) in acetonitrile (100 ml) was refluxed for 5h.

The reaction mixture was concentrated under reduced pressure. The residue was chromatographed using a silica gel column (chloroform/methanol=6/1 v/v). 6-[N-{p-(2-hydroxy-3-isopropylaminopropoxy)phenyl}amino]hexyl 1,4-dihydro-3-methoxycarbonyl-6-methyl-4-m-nitrophenyl-2-trifluoromethylpyridine-5-carboxylate was obtained as a yellow oil (530 mg).

To a solution of 6-[N-{p-(2-hydroxy-3-isopropylaminopropoxy)phenyl}amino]hexyl 1,4-dihydro-3-methoxycarbonyl-6-methyl-4-m-nitrophenyl-2-trifluoromethylpyridine-5-carboxylate in methanol (3.0 ml) was added 2N methanol solution of hydrogen chloride (2.0 ml). The reaction mixture was stirred for 1 h at room temperature. Methanol was evaporated under reduced pressure. 6-[N-{p-(2-aydroxy-3-isopropylaminopropoxy)phenyl}amino]hexyl 1,4-dihydro-3-methoxycarbonyl-6-methyl-4-m-nitrophenyl-2-trifluoromethylpyridine-5-carboxylate dihydrochloride as a yellow stiff foam was obtained as a yellow stiff foam (280 mg).
IR (KBr) 3344, 2950, 2863, 1703, 1624, 1582, 1530, 1513, 1437, 1387, 1349, 1281, 1256, 1224, 1178, 1085, 829, 756 cm⁻¹;
¹H-NMR (CD₃OD-TMS) δ 8.12-8.02 (m, 2H), 7.66-7.62 (m, 1H), 7.53 (t, 1H), 7.49-7.45 (m, 2H), 7.20-7.15 (m, 2H), 5.06 (s, 1H), 4.32-4.25 (m, 1H), 4.17-3.96 (m, 4H), 3.68 (s, 3H), 3.53-3.10 (m, 5H), 2.42 (s, 3H), 1.71-1.55 (m, 4H), 1.47-1.18 (m, 10H);
mass spectrum, m/e (FD) 693 (M⁺+1).

### Example 51

Preparation of 6-[N-{p-(2-hydroxy-3-isopropylaminopropoxy)phenyl}amino]hexyl 2-cyano-1,4-dihydro-3-methoxycarbonyl-6-methyl-4-m-nitrophenylpyridine-5-carboxylate dihydrochloride.

The procedure described in Example 50 was followed using 6-bromohexyl 2-cyano-1,4-dihydro-3-methoxycarbonyl-6-methyl-4-m-nitrophenylpyridine-5-carboxylate (980 mg) and p-(2-hydroxy-3-isopropylaminopropoxy)aniline (2160 mg) as starting materials. 6-[N-{p-(2-Hydroxy-3-isopropylaminopropoxy) phenyl}amino]hexyl 2-cyano-1,4-dihydro-3-methoxycarbonyl-6-methyl-4-m-nitrophenyl pyridine-5-carboxylate dihydrochloride as a yellow stiff foam (200 mg).
IR (KBr) 3358, 3180, 3076, 2950, 2236, 1704, 1529, 1512, 1462, 1386, 1349, 1256, 1218, 1099 cm⁻¹;
¹H-NMR (CD₃OD-TMS) δ 8.10-8.01 (m, 2H), 7.68-7.63 (m, 1H), 7.53 (t, 1H), 7.47-7.42 (m, 2H), 7.19-7.13 (m, 2H), 5.17 (s, 1H), 4.31-4.24 (m, 1H), 4.16-3.93 (m, 4H), 3.74 (s, 3H), 3.55-3.10 (m, 5H), 2.38 (s, 3H), 1.71-1.53 (m, 4H), 1.45-1.10 (m, 10H);
mass spectrum, m/e (Fd) 650 (M⁺+1).

### Example 52

Preparation of 3-[N-{p-(2-hydroxy-3-isopropylaminopropoxy)phenyl}amino]propyl 1,4-dihydro-3-methoxycarbonyl-6-methyl-4-m-nitrophenyl-2-trifluoromethylpyridine-5-carboxylate dihydrochloride.

The procedure described in Example 50 was followed using 3-bromopropyl 1,4-dihydro-3-methoxycarbonyl-6-methyl-4-m-nitrophenyl-2-trifluoromethylpyridine-5-carboxylate (1290 mg) and p-(2-hydroxy-3-isopropylaminopropoxy)aniline (2000 mg) as starting materials. 3-[N-{p-(2-Hydroxy-3-isopropylaminopropoxy)phenyl}amino]propyl 1,4-dihydro-3-methoxycarbonyl-6-methyl-4-m-nitrophenyl-2-trifluoromethylpyridine-5-carboxylate dihydrochloride as a yellow stiff foam (370 mg).
IR (KBr) 3350, 2953, 2742, 1706, 1623, 1530, 1513, 1437, 1386, 1351, 1282, 1255, 1224, 1178, 1086, 830 cm⁻¹;
¹H-NMR (CD₃OD-TMS) δ 8.07-7.98 (m, 2H), 7.65-7.58 (m, 1H), 7.51 (t, 1H), 7.38-7.30 (m, 2H), 7.19-7.10 (m, 2H), 5.05 (s, 1H), 4.32-4.03 (m, 5H), 3.69 (s, 3H), 3.53-3.08 (m, 5H), 2.45 (s, 3H), 2.07-1.93 (m, 2H), 1.38 (t, 6H);
mass spectrum m/e (FD) 651 (M⁺+1).

### Example 53

Preparation of 2-[N-{p-(2-hydroxy-3-isopropylaminopropoxy)phenyl}-N-methylamino]ethyl 1,4-dihydro-3-methoxycarbonyl-6-methyl-4-m-nitrophenyl-2-trifluoromethylpyridine-5-carboxylate dihydrochloride

The procedure described in Example 38 was followed using 1,4-dihydro-3-methoxycarbonyl-6-methyl-4-m-nitrophenyl-2-trifluoromethylpyridine-5-carboxylic acid (1030 mg), 2-[N-{p-(2-hydroxy-3-isopropylaminopropoxy)}phenyl-N-methylamino]ethanol (1110 mg), N,N'-dicyclohexylcarbodiimide (830 mg) and 4-dimethylaminopyridine (370 mg) as starting materials. 2-[N-{p-(2-Hydroxy-3-isopropylaminopropoxy)phenyl}-N-methylamino]ethyl 1,4-dihydro-3-methoxycarbonyl-6-methyl-4-m-nitrophenyl-2-trifluoromethylpyridine-5-carboxylate dihydrochloride was obtained as a paleyellow stiff foam (330 mg).
IR (KBr) 3358, 2958, 1709, 1619, 1530, 1514, 1461, 1438, 1386, 1351, 1280, 1257, 1224, 1182, 1086, 1039, 756 cm⁻¹;
¹H-NMR (CD₃COCD₃-TMS) δ 8.70 (d, 1H), 8.14 (s, 1H), 8.09 (d, 1H), 8.01 (s, 1H), 7.80-7.70 (m, 1H), 7.76 (d, 2H), 7.24 (t, 1H), 7.04 (d, 2H), 5.16 (s, 1H), 4.54-4.44 (m, 2H), 4.30-4.22 (m, 1H), 4.18-4.06 (m, 2H), 3.80 (br s, 2H), 3.72 (s, 3H), 3.62 (t, 1H), 3.60-3.52 (m, 2H), 3.11 (s, 3H), 2.37 (s, 3H), 1.49 (d, 6H);
mass spectrum, m/e (Fd) 651 (M⁺+1).

### Example 54

Preparation of 4-[N-{p-(2-hydroxy-3-isopropylaminopropoxy)phenyl}amino]butyl 2-cyano-1,4-dihydro-3-methoxycarbonyl-6-methyl-4-m-nitrophenylpyridine-5-carboxylate.

The procedure described in Example 50 was followed using 4-bromobutyl 2-cyano-1,4-dihydro-3-methoxycarbonyl-6-methyl-4-m-nitrophenylpyridine-5-carboxylate (1440 mg) and p-(2-hydroxy-3-isopropylaminogropoxy)aniline (3050 mg) as starting materials. 4-[N-{p-(2-Hydroxy-3-isopropylaminopropoxy) pheny}amino]butyl 2-cyano 1,4-dihydro-3-methoxycarbonyl-6-methyl-4-m-nitrophenyl pyridine-5-carboxylate as an orange stiff foam (660 mg).
IR (KBr) 3186, 3078, 2957, 2236, 1705, 1530, 1513, 1476, 1385, 1349, 1216, 1097 cm⁻¹;
¹H-NMR (CDCl₃-TMS ) δ 8.11-8.03 (m, 2H), 7.60 (d, 1H), 7.41 (t, 1H), 6.81-6.72 (m, 2H), 6.55-6.48 (m, 2H), 5.163 and 5.157 (2s, 1H), 4.21-3.90 (m, 5H), 3.77 (s, 3H), 3.18-2.77 (m, 5H), 2.274 and 2.262 (2s, 3H), 1.74-1.46 (m, 4H), 1.156 (d, 6H);
mass spectrum, m/e (FD) 622 (M⁺+1).

### Example 55

Preparation of 2-[N-{p-(2-hydroxy-3-isopropylaminopropoxy)phenyl}-N-methylamino]ethyl 2-cyano-1,4-dihydro-3-methoxycarbonyl-6-methyl-4-m-nitrophenylpyridine-5-carboxylate.

The procedure described in Example 38 was followed using 2-cyano-1,4-dihydro-3-methoxycarbonyl -6-methyl-4-m-nitrophenylpyridine-5-carboxylic acid (860 mg), 2-[N-{p-(2-hydroxy-3-isopropylaminopropoxy)}phenyl-N-methylamino]ethanol (940 mg), N,N'-dicyclohexyl-carbodiimide (770 mg) and 4-dimethylaminopyridine (340 mg) as starting materials. 2-[N-{(2-Hydroxy-3-isopropylaminopropoxy)phenyl}-N-methylamino]ethyl 2-cyano-1,4-dihydro-3-methoxycarbonyl-6-methyl-4-m-nitrophenylpyridine-5-carboxylate was obtained as a pale yellow stiff foam (290 mg).
IR (KBr) 3294, 3078, 2964, 2236, 1705, 1514, 1472, 1436, 1349, 1310, 1298, 1271, 1214, 1117, 1098, 1036, 753 cm⁻¹;
¹H-NMR (CDCl₃-TMS ) δ 8.08-8.03 (m, 3H), 7.56 (t, 1H), 7.41 (dd, 1H), 6.75 (dd, 2H), 6.51 (dd, 2H), 5.09 and 5.07 (2s, 1H), 4.40-3.90 (m, 5H), 3.783 and 3.778 (2s, 3H), 3.51 (dd, 2H), 2.95-2.87 (m, 2H), 2.83 and 2.80 (2s, 3H), 2.81-2.75 (m, 1H), 2.19 and 2.18 (2s, 3H), 1.17-1.12 (m, 6H);
mass spectrum, m/e (FD) 608 (M⁺+1).

### Example 56

Preparation of p-(2-hydroxy-3-isopropylaminopropoxy)benzyl 1,4-dihydro-3-methoxycarbonyl-6-methyl-2-trifluoromethyl-4-m-trifluorophenylpyridine-5-carboxylate monohydrochloride.

The procedure described in Example 5 was followed using p-(2,3-epoxypropoxy)benzyl 1,4-dihydro-3-methoxycarbonyl-6-methyl-2-trifluoromethyl-4-m-trifluorophenylpyridine-5-carboxylate (730 mg) and isopropylamine (0.8 ml) as starting materials. p-(2-Hydroxy-3-isopropylaminopropoxy)benzyl 1,4-dihydro-3-methoxycarbonyl-6-methyl-2-trifluoromethyl-4-m-trifluorophenylpyridine-5-carboxylate monohydrochloride was obtained as a pale yellow stiff foam (560 mg).
IR (KBr) 3350, 2956, 1706, 1616, 1515, 1438, 1386, 1329, 1281, 1220, 1178, 1125, 1096, 1074, 982 828 790 754 cm⁻¹;
¹H-NMR (CDCl₃-TMS) δ 7.44-7.30 (m, 4H), 7.07 (d, 2H), 6.80 (d, 2H), 6.19 (br s, 1H), 5.065 (s, 1H), 5.01 (d, 1H), 4.59 (d, 1H), 4.08-4.04 (m, 1H), 3.99-3.95 (m, 1H), 3.650 (s, 3H), 3.45-3.40 (m, 1H), 3.31-3.26 (m, 1H), 3.17-3.10 (m, 1H), 2.381 (s, 3H), 1.48-1.43 (m, 6H);
mass spectrum, m/e (FD)631 (M⁺+1).

The structural formulae of the compounds prepared in Examples 16 to 56 are as follows:

### Example 16

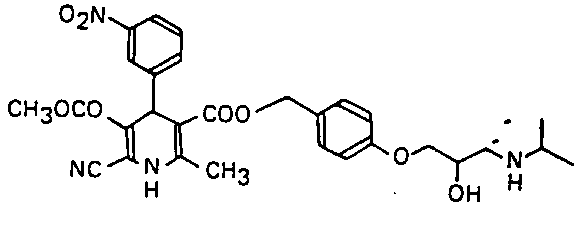

### Example 17

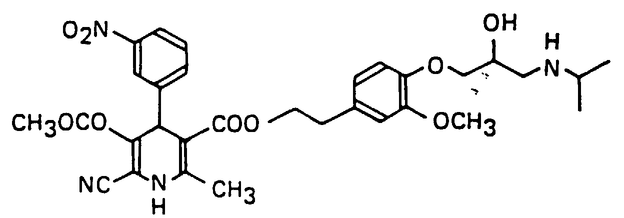

### Example 18

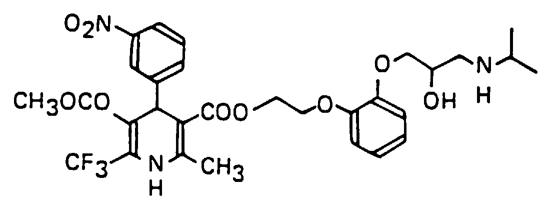

### Example 19

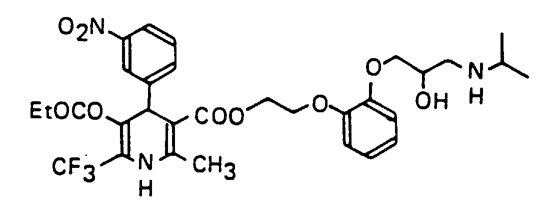

### Example 20

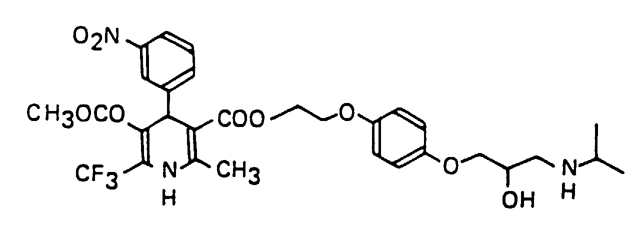

### Example 21

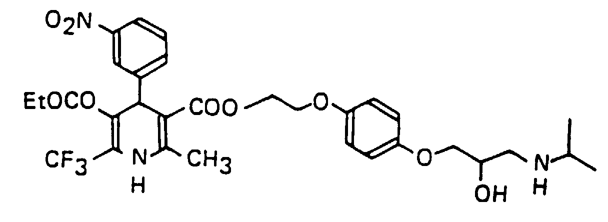

### Example 22

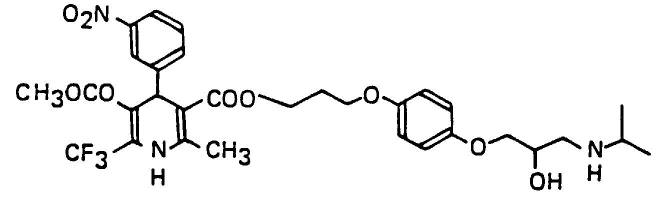

### Example 23

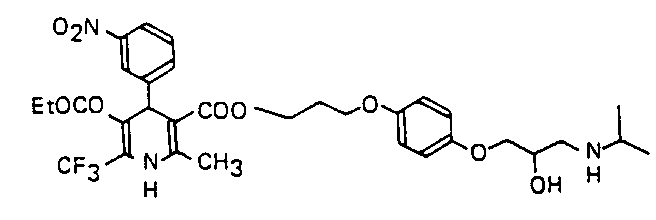

### Example 24

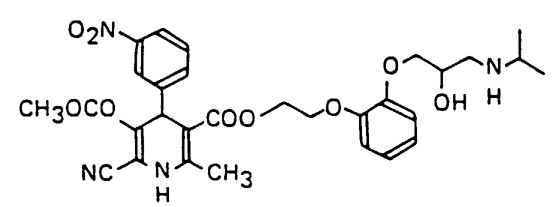

### Example 25

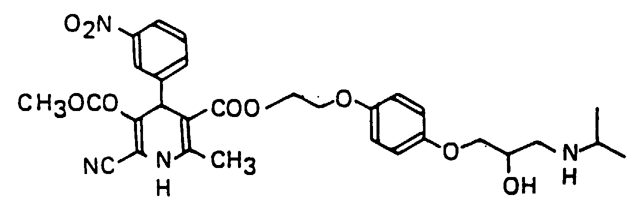

### Example 26

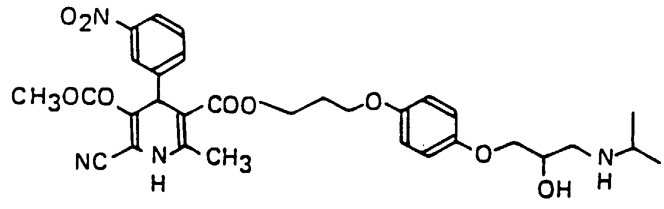

### Example 27

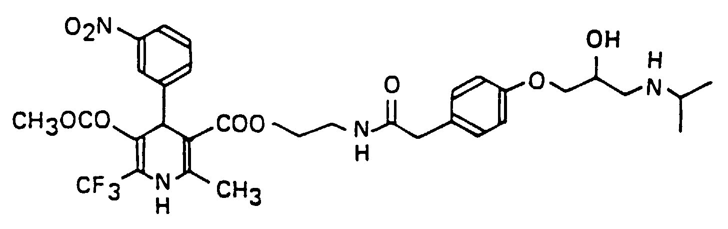

### Example 28

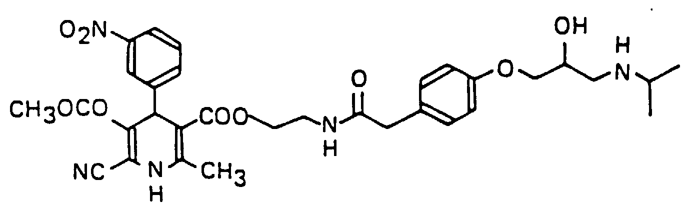

### Example 29

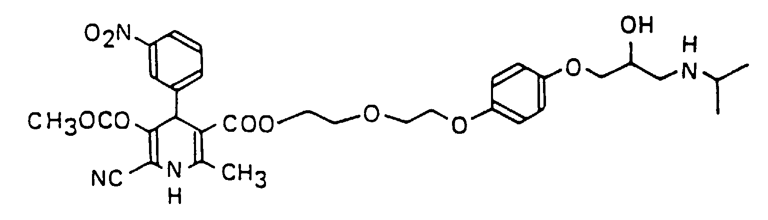

### Example 30

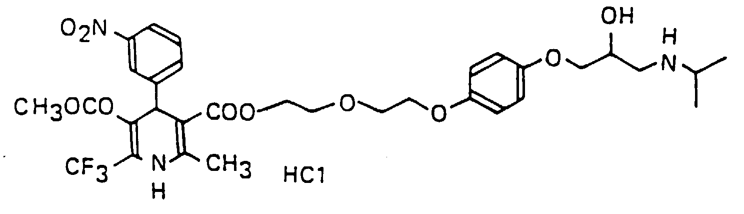

### Example 31

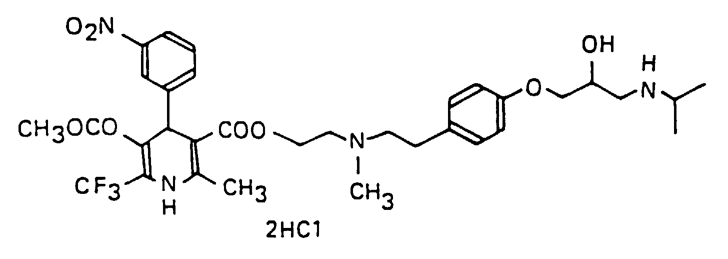

### Example 32

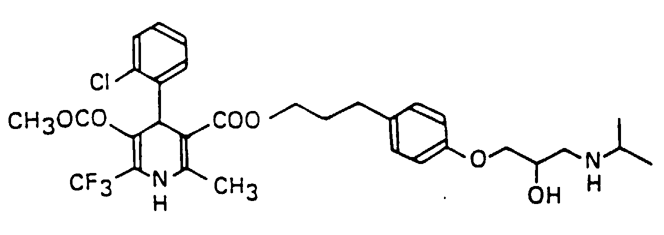

### Example 33

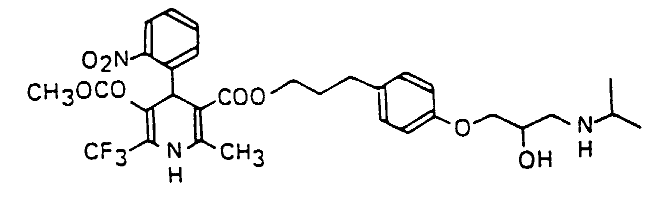

### Example 34

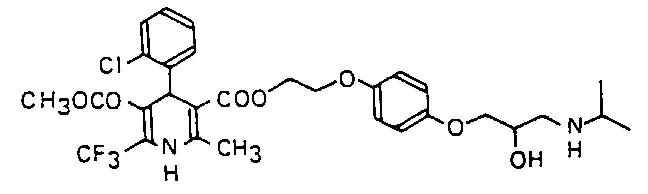

### Example 35

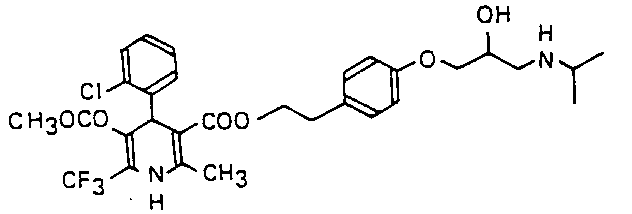

### Example 36

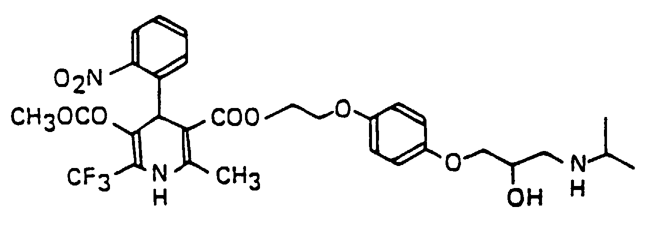

### Example 37

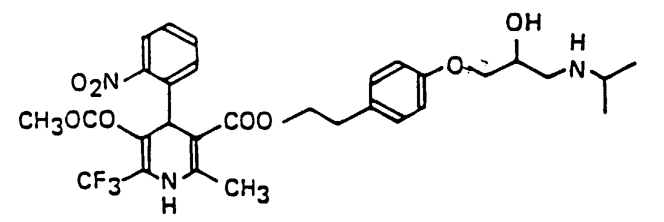

### Example 38

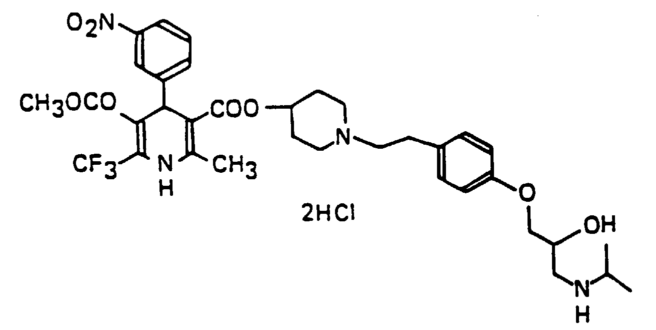

### Example 39

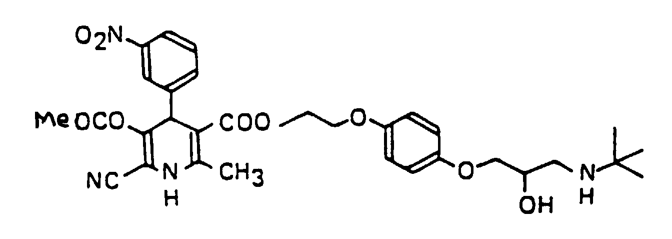

### Example 40

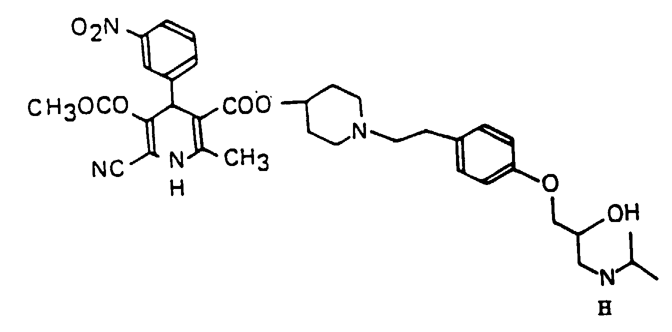

### Example 41

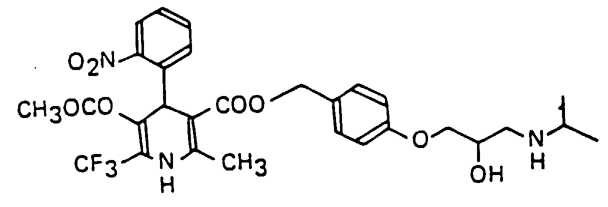

### Example 42

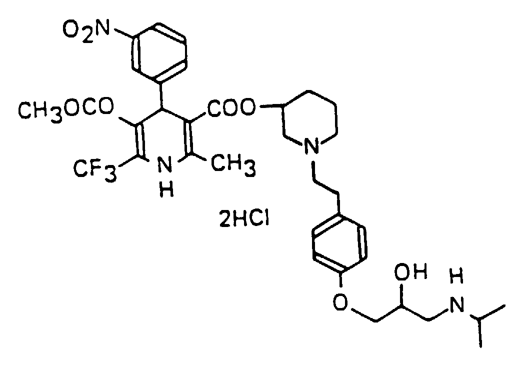

### Example 43

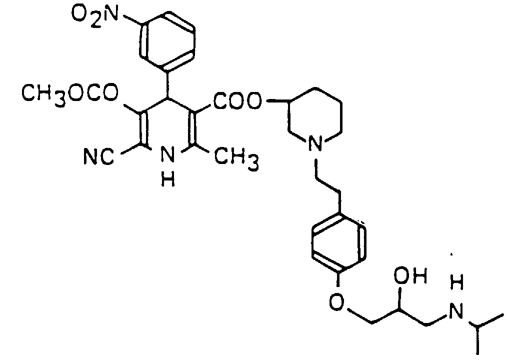

### Example 44

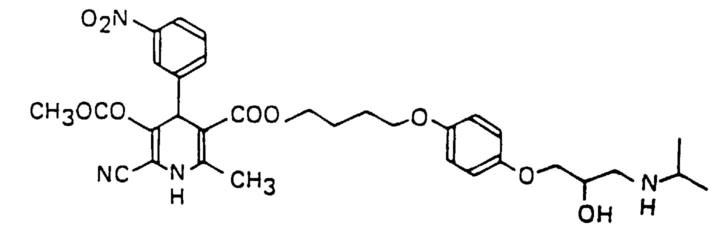

### Example 45

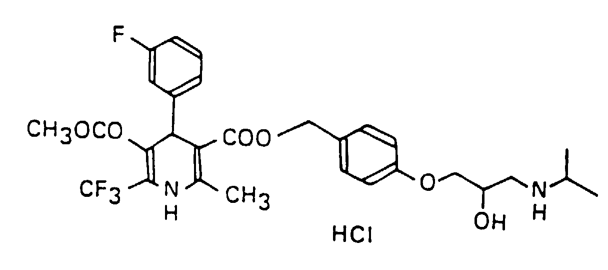

### Example 46

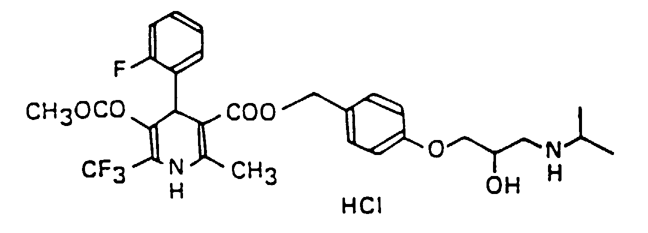

### Example 47

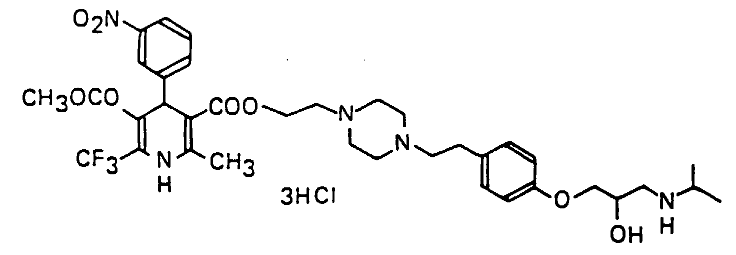

### Example 48

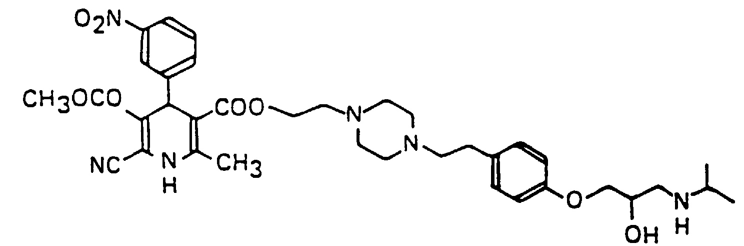

### Example 49

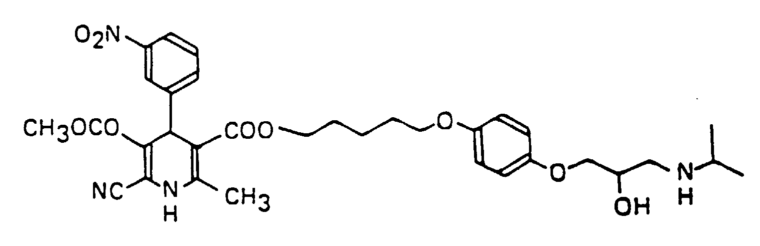

### Example 50

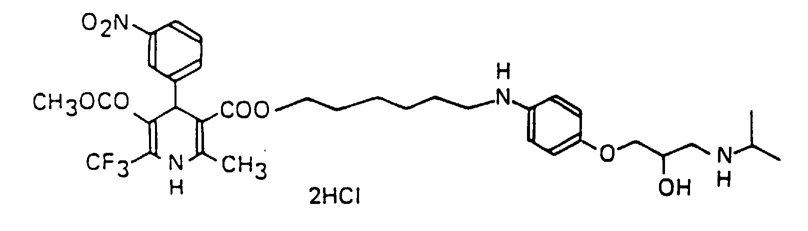

### Example 51

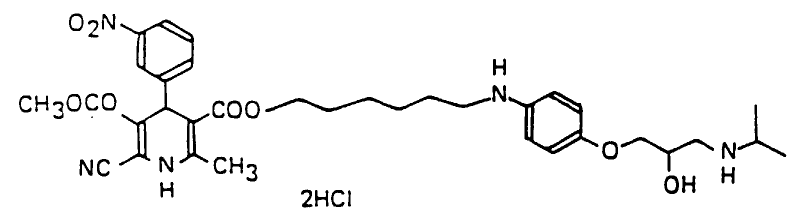

### Example 52

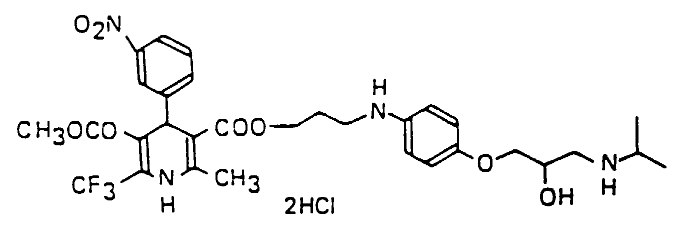

### Example 53

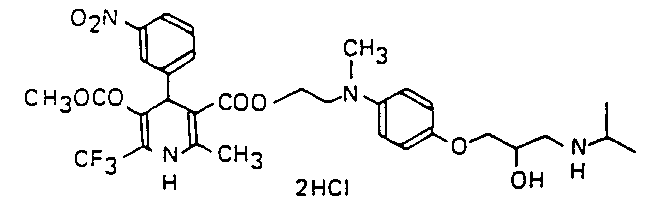

### Example 54

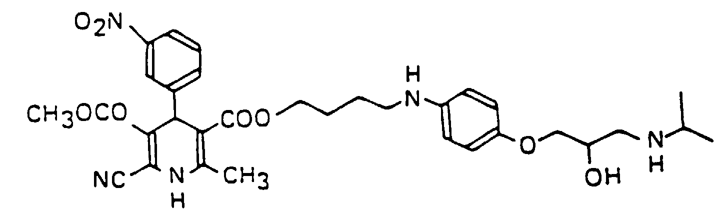

### Example 55

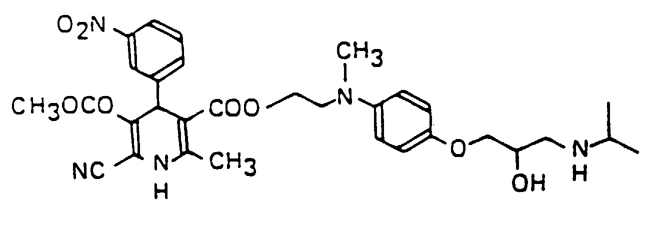

### Example 56

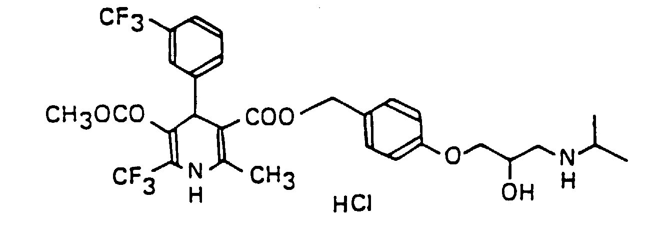

## Claims

1. A dihydropyridine compound of the formula: 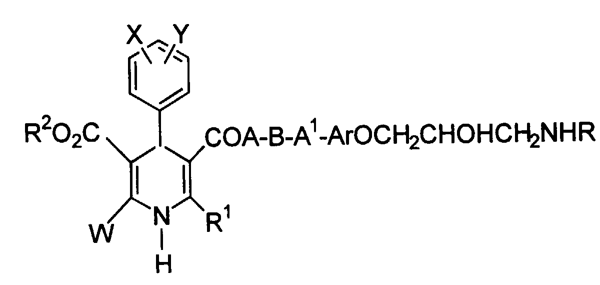 wherein W represents a cyano or halo alkyl group the alkyl group of which is straight chained or branched and contains 1 to 6 carbon atoms; R, R¹ and R², which may be the same or different, each represent a saturated hydrocarbon group which is straight chained or branched and which contains 1 to 6 carbon atoms; X and Y, which may be the same or different, each represents a hydrogen atom, a halogen atom, a halo alkyl group in which the alkyl group is straight chained or branched and contains 1 to 6 carbon atoms, or a nitro group; Ar represents a phenylene group which may contain a methoxy substituent; A represents -O- or -NH-; B represents a direct link or a straight chained or branched bivalent paraffinic hydrocarbon residue of 1 to 10 carbon atoms and A¹ represents a direct link, oxa, imino, an alkylimino having 1 to 6 carbon atoms, amido, an alkylamido having 1 to 6 carbon atoms, oxyalkylenoxy group, a lower or higher straight chained or branched bivalent paraffinic hydrocarbon residue having respectively 2 to 6 or 7 to 12 carbon atoms optionally interrupted by oxa, imino, an alkylimino having 1 to 6 carbon atoms, amido, an alkylamido having 1 to 6 carbon atoms or a saturated or unsaturated, 3 to 8-membered heteromonocylic group containing 1 to 4 nitrogen atoms and optionally 1 or 2 oxygen atoms, or a group of the formula; -Het-Q-(wherein Het is a bivalent, saturated or unsaturated, 3 to 8-membered heteromonocyclic group containing 1 to 4 nitrogen atoms and optionally 1 or 2 oxygen atoms and Q is a direct link or a straight-chained or branched bivalent paraffinic hydrocarbon residue having 2 to 6 carbon atoms); or acid addition salts thereof.

2. A dihydropyridine compound claimed in claim 1 characterised in that each halogen atom is fluorine, chlorine or bromine.

3. A dihydropyridine compound claimed in claim 1 or 2 in which the acid addition salt is a hydrochloride or sulphate.

4. A dihydropyridine compound claimed in claim 1 or 2 in which the acid addition salt is an oxalate, lactate, succinate, tartarate, maleate, fumarate, acetate, salicylate, citrate, benzoate betanaphthoate, adipate, methanesulphonate, benzenesulphonate or p-toluenesulphonate.

5. A process for preparing a dihydropyridine compound claimed in claim 1 characterised in that it comprises reacting an amine of the formula:
RNH₂
wherein R has the meanings defined in claim 1, with an epoxide or an alcohol derivative of the respective formulae: 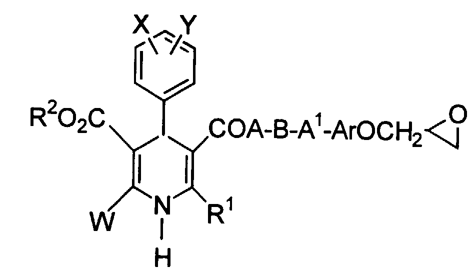 and 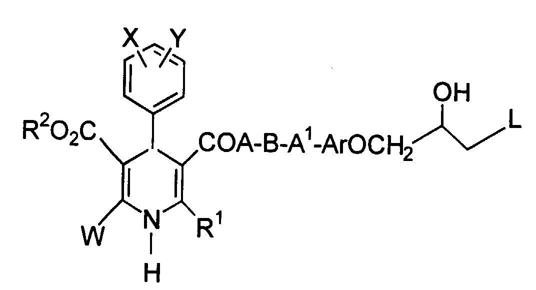 wherein W, R¹, R², R, A, B, A¹, Ar, X, and Y have the meanings defined in claim 1, and L stands for a suitable leaving group(reactive functional group).

6. A process for preparing a dihydropyridine compound claimed in claim 1 characterised in that it comprises reacting a dihydropyridine derivative of the formula: 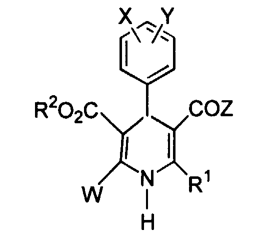 wherein W, R¹, R², X and Y have the meanings defined in claim 1 and wherein Z stands for a suitable leaving group(reactive functional group), with a compound of the formula:
H-A-B-A¹-ArOCH₂CHOHCH₂NHR
wherein R, A, B, A¹, and Ar have the meanings defined in claim 1.

7. A process for preparing a dihydropyridine compound claimed in claim 1 characterised in that it comprises reacting a dihydropyridine derivative of the formula: 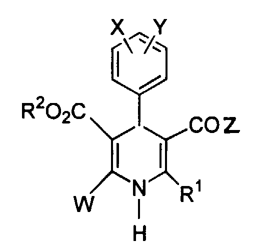 wherein W, R¹, R², X and Y have the meanings defined in claim 1 and wherein Z stands for a suitable leaving group(reactive functional group), with a compound of the formula: 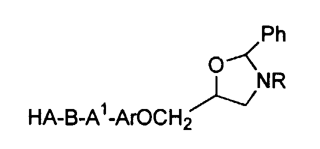 wherein R, A, B, A¹, and Ar have the meanings defined in claim 1 and wherein Ph represents a phenyl group, and successively removing the N,O-benzylidene acetal.

8. A process for preparing a dihydropyridine compound claimed in claim 1 characterised in that it comprises reacting a dihydropyridine derivative of the formula: 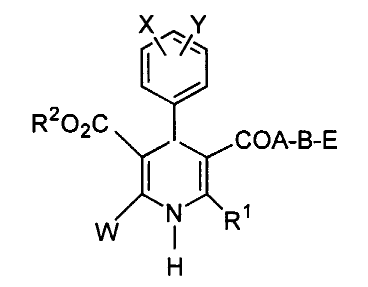 wherein W, R¹, R², X, Y, A, and B have the meanings defined in claim 1 and wherein E stands for a suitable leaving group(reactive functional group), with a compound of the formula:
H₂NArOCH₂CHOHCH₂NHR
wherein R and Ar have the meanings defined in claim 1.

9. A process for manufacturing the dihydropyridine derivatives claimed in claim 1 characterised in that it comprises the reduction of an aminoketone of the formula: 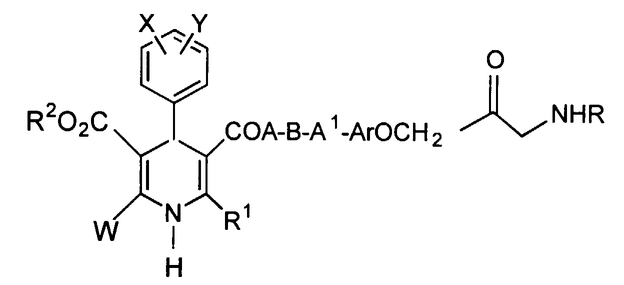 wherein W, R¹, R², R, A, B, A¹, Ar, X, and Y have the meanings defined in claim 1.

## Patentansprüche

1. Dihydropyridinverbindung der Formel: 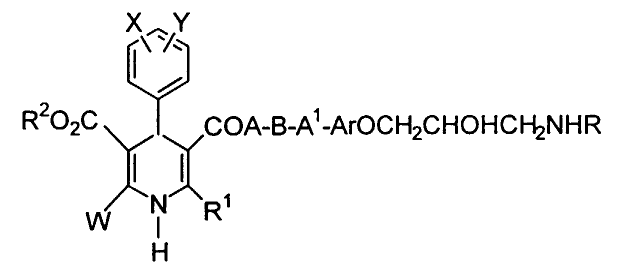 worin W eine Cyano- oder Halogenalkylgruppe darstellt, deren Alkylgruppe unverzweigt oder verzweigt ist und 1 bis 6 C-Atome enthält, R, R¹ und R², welche gleich oder unterschiedlich sein können, jeweils eine gesättigte Kohlenwasserstoffgruppe, die unverzweigt oder verzweigt ist und 1 bis 6 C-Atome enthält, bedeuten, X und Y, welche gleich oder unterschiedlich sein können, jeweils ein Wasserstoffatom, ein Halogenatom, eine Halogenalkylgruppe, in der die Alkylgruppe unverzweigt oder verzweigt ist und 1 bis 6 C-Atome enthält, oder eine Nitrogruppe darstellen, Ar eine Phenylengruppe, welche einen Methoxysubstituenten enthalten kann, bedeutet, A -O- oder -NH- darstellt, B eine Direktbindung oder einen unverzweigten oder verzweigten zweiwertigen Paraffinkohlenwasserstoffrest mit 1 bis 10 C-Atomen bedeutet und A' eine Direktbindung, eine Oxa-, eine Imino-, eine Alkyliminogruppe mit 1 bis 6 C-Atomen, eine Amido-, eine Alkylamidogruppe mit 1 bis 6 C-Atomen, eine Oxyalkylenoxygruppe, einen niederen oder höheren unverzweigten oder verzweigten zweiwertigen Paraffinkohlenwasserstoffrest mit jeweils 2 bis 6 oder 7 bis 12 C-Atomen, der gegebenenfalls durch eine Oxa-, eine Iminogruppe, eine Alkyliminogruppe mit 1 bis 6 C-Atomen, eine Amido-, eine Alkylamidogruppe mit 1 bis 6 C-Atomen unterbrochen ist, oder eine gesättigte oder ungesättigte, 3- bis 8-gliedrige heteromonocyclische Gruppe, welche 1 bis 4 Stickstoffatome oder gegebenenfalls 1 oder 2 Sauerstoffatome enthält, oder eine Gruppe der Formel -Het-Q, worin Het eine zweiwertige gesättigte oder ungesättigte 3-bis 8-gliedrige heteromonocyclische Gruppe, welche 1 bis 4 Stickstoffatome oder gegebenenfalls 1 oder 2 Sauerstoffatome enthält, bedeutet und Q eine Direktbindung oder einen unverzweigten oder verzweigten zweiwertigen Paraffinkohlenwasserstoffrest mit 2 bis 6 C-Atomen darstellt, oder Säureadditionssalze davon.

2. Dihydropyridinverbindung nach Anspruch 1, dadurch gekennzeichnet, daß jedes Halogenatom Fluor, Chlor oder Brom ist.

3. Dihydropyridinverbindung nach Anspruch 1 oder 2, wobei das Säureadditionssalz ein Hydrochlorid oder Sulfat ist.

4. Dihydropyridinverbindung nach Anspruch 1 oder 2, wobei das Säureadditionssalz ein Oxalat, Lactat, Succinat, Tartrat, Maleat, Fumarat, Acetat, Salicylat, Citrat, Benzoat, β-Naphthoat, Adipat, Methansulfonat, Benzolsulfonat oder p-Toluolsulfonat ist.

5. Verfahren zur Herstellung einer Dihydropyridinverbindung nach Anspruch 1, dadurch gekennzeichnet, daß ein Amin der Formel:
RNH₂
worin R die in Anspruch 1 angegebenen Bedeutungen hat, mit einem Epoxid oder einem Alkoholderivat der Formeln 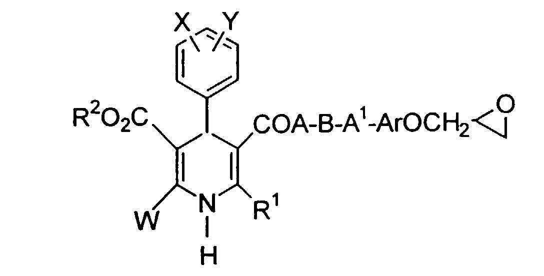 und 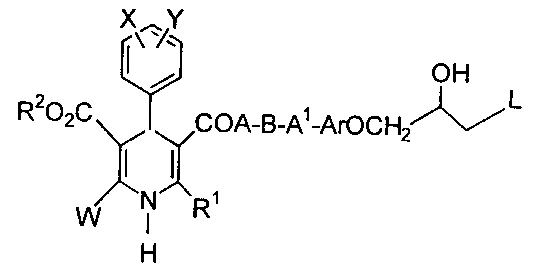 worin W, R¹, R², R, A, B, A¹, Ar, X und Y die in Anspruch 1 angegebenen Bedeutungen haben und L für eine geeignete Austrittsgruppe (reaktionsfähige funktionelle Gruppe) steht, umgesetzt wird.

6. Verfahren zur Herstellung einer Dihydropyridinverbindung nach Anspruch 1, dadurch gekennzeichnet, daß ein Dihydropyridinderivat der Formel: 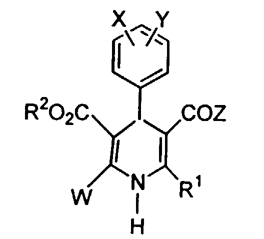 worin W, R¹, R², X und Y die in Anspruch 1 angegebenen Bedeutungen haben und Z für eine geeignete Austrittsgruppe (reaktionsfähige funktionelle Gruppe) steht, mit einer Verbindung der Formel:
H-A-B-A¹-ArOCH₂CHOHCH₂NHR
worin R, A, B, A¹ und Ar die in Anspruch 1 angegebenen Bedeutungen haben, umgesetzt wird.

7. Verfahren zur Herstellung einer Dihydropyridinverbindung nach Anspruch 1, dadurch gekennzeichnet, daß ein Dihydropyridinderivat der Formel: 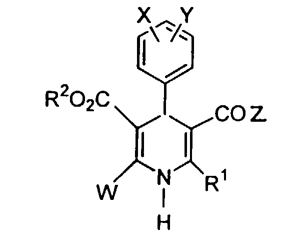 worin W, R¹, R², X und Y die in Anspruch 1 angegebenen Bedeutungen haben und Z für eine geeignete Austrittsgruppe (reaktionsfähige funktionelle Gruppe) steht, mit einer Verbindung der Formel: 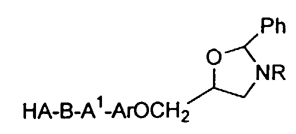 worin R, A, B, A¹ und Ar die in Anspruch 1 angegebenen Bedeutungen haben und Ph eine Phenylgruppe darstellt, umgesetzt wird, wonach das N,O-Benzylidenacetal abgetrennt wird.

8. Verfahren zur Herstellung einer Dihydropyridinverbindung nach Anspruch 1, dadurch gekennzeichnet, daß ein Dihydropyridinderivat der Formel: 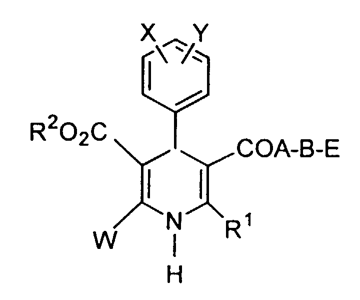 worin W, R¹, R², X, Y, A und B die in Anspruch 1 angegebenen Bedeutungen haben und E für eine geeignete Austrittsgruppe (reaktionsfähige funktionelle Gruppe) steht, mit einer Verbindung der Formel:
H₂NArOCH₂CHOHCH₂NHR
worin R und Ar die in Anspruch 1 angegebenen Bedeutungen haben, umgesetzt wird.

9. Verfahren zur Herstellung des Dihydropyridinderivats nach Anspruch 1, dadurch gekennzeichnet, daß es die Reduktion eines Aminoketons der Formel: 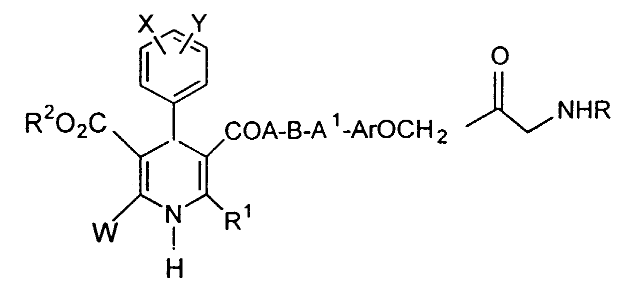 worin W, R¹, R², R, A, B, A¹, Ar, X und Y die in Anspruch 1 angegebenen Bedeutungen haben, umfaßt.

## Revendications

1. Composé dihydropyridine de formule: 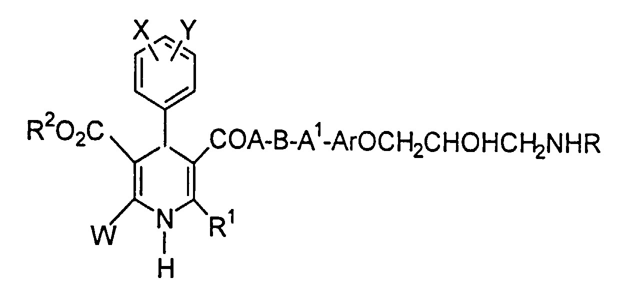 dans laquelle W représente un groupe cyanoalkyle ou halogénoalkyle, dont le groupe est à chaîne linéaire ou ramifiée et contient 1 à 6 atomes de carbone; R, R¹ et R², qui peuvent être identiques ou différents, représentent chacun un groupe hydrocarboné saturé, qui est à chaîne linéaire ou ramifiée et qui contient 1 à 6 atomes de carbone; X et Y, qui peuvent être identiques ou différents, représentent chacun un atome d'hydrogène, un atome d'halogène, un groupe halogénoalkyle dans lequel le groupe alkyle est à chaîne linéaire ou ramifiée et contient 1 à 6 atomes de carbone, ou un groupe nitro; Ar représente un groupe phénylène qui peut contenir un substituant méthoxy; A représente -O- ou -NH-; B représente une liaison directe ou un résidu hydrocarboné paraffinique bivalent, à chaîne linéaire ou ramifiée, de 1 à 10 atomes de carbone, et A¹ représente une liaison directe, un groupe oxa, un groupe imino, un groupe alkylimino comportant 1 à 6 atomes de carbone, un groupe amido, un groupe alkylamido comportant 1 à 6 atomes de carbone, un groupe oxyalkylèneoxy, un résidu hydrocarboné paraffinique bivalent, inférieur ou supérieur à chaîne linéaire ou ramifiée comportant respectivement 2 à 6 ou 7 à 12 atomes de carbone, éventuellement interrompu par un groupe oxa, un groupe imino, un groupe alkylimino comportant 1 à 6 atomes de carbone, un groupe amido, un groupe alkylamido comportant 1 à 6 atomes de carbone ou un groupe hétéromonocyclique à 3 à 8 chaînons, saturé ou insaturé, contenant 1 à 4 atomes d'azote et, éventuellement, 1 ou 2 atomes d'oxygène, ou un groupe de formule -Het-Q-(dans lequel Het est un groupe hétéromonocyclique bivalent à 3 à 8 chaînons, saturé ou insaturé, contenant 1 à 4 atomes d'azote et, éventuellement, 1 ou 2 atomes d'oxygène, et Q est une liaison directe ou un résidu hydrocarboné paraffinique bivalent, à chaîne linéaire ou ramifiée comportant 2 à 6 atomes de carbone; ou ses sels d'addition d'acide.

2. Composé dihydropyridine selon la revendication 1, caractérisé en ce que chaque atome d'halogène est un atome de fluor, de chlore ou de brome.

3. Composé dihydropyridine selon la revendication 1 ou 2, dans lequel le sel d'addition d'acide est un chlorhydrate ou un sulfate.

4. Composé dihydropyridine selon la revendication 1 ou 2, dans lequel le sel d'addition d'acide est un oxalate, un lactate, un succinate, un tartrate, un maléate, un fumarate, un acétate, un salicylate, un citrate, un benzoate, un β-naphtoate, un adipate, un méthanesulfonate, un benzènesulfonate ou un p-toluènesulfonate.

5. Procédé de préparation d'un composé dihydropyridine selon la revendication 1, caractérisé en ce qu'il comprend la réaction d'une amine de formule:
RNH₂
dans laquelle R a la signification définie dans la revendication 1, avec un époxyde ou un dérivé d'alcool, de formules respectives: 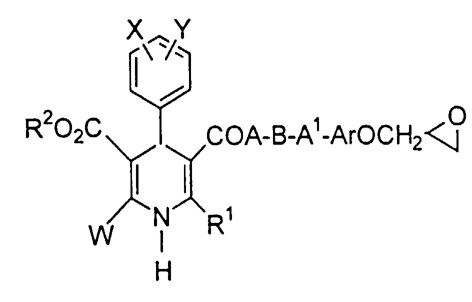 et 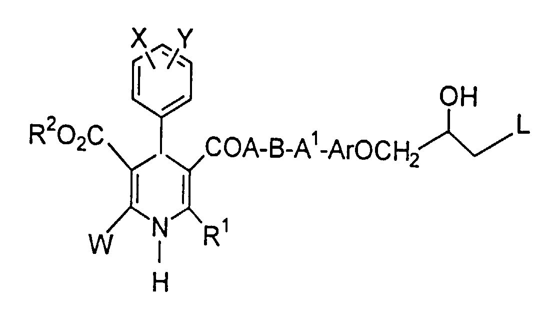 dans lesquelles W, R¹, R², R, A, B, A¹, Ar, X et Y ont les significations définies dans la revendication 1, et L représente un groupe partant convenable (groupe fonctionnel réactif).

6. Procédé de préparation d'un composé dihydropyridine selon la revendication 1, caractérisé en ce qu'il comprend la réaction d'un dérivé de dihydropyridine de formule: 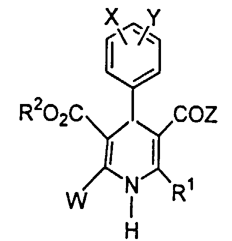 dans laquelle W, R¹, R², X et Y ont les significations définies dans la revendication 1, et dans laquelle Z représente un groupe partant convenable (groupe fonctionnel réactif), avec un composé de formule:
H-A-B-A¹-ArOCH₂CHOHCH₂NHR
dans laquelle R, A, B, A¹ et Ar ont les significations définies dans la revendication 1.

7. Procédé de préparation d'un composé dihydropyridine selon la revendication 1, caractérisé en ce qu'il comprend la réaction d'un dérivé de dihydropyridine de formule: 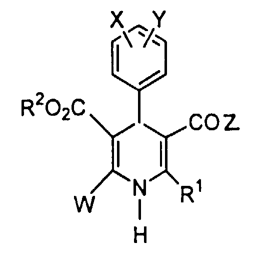 dans laquelle W, R¹, R², X et Y ont les significations définies dans la revendication 1, et dans laquelle Z représente un groupe partant convenable (groupe fonctionnel réactif), avec un composé de formule: 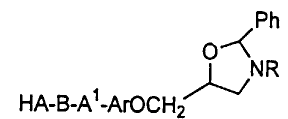 dans laquelle R, A, B, A¹ et Ar ont les significations définies dans la revendication 1, et dans laquelle Ph représente un groupe phényle, suivie de l'élimination du N,O-benzylidène-acétal.

8. Procédé de préparation d'un composé dihydropyridine selon la revendication 1, caractérisé en ce qu'il comprend la réaction d'un dérivé de dihydropyridine de formule: 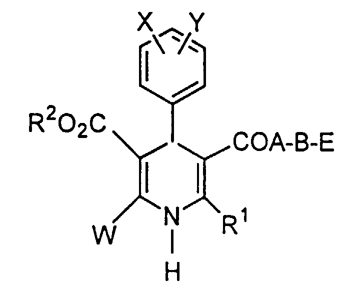 dans laquelle W, R¹, R², X, Y, A et B ont les significations définies dans la revendication 1 et dans laquelle E représente un groupe partant convenable (groupe fonctionnel réactif), avec un composé de formule:
H₂NArOCH₂CHOHCH₂NHR
dans laquelle R et Ar ont les significations définies dans la revendication 1.

9. Procédé de préparation des dérivés de dihydropyridine selon la revendication 1, caractérisé en ce qu'il comprend la réduction d'une aminocétone de formule: 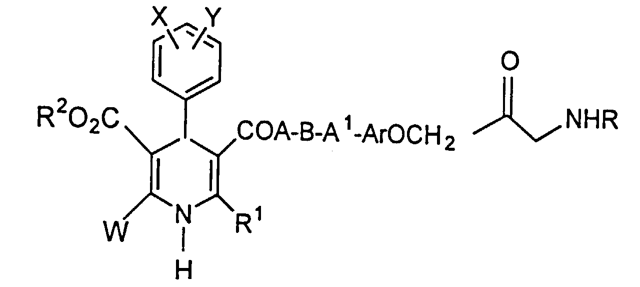 dans laquelle W, R¹, R², R, A, B, A¹, Ar, X et Y ont les significations définies dans la revendication 1.
